# EUROPEAN PATENT APPLICATION

(11) **EP 4 678 234 A1**
(43) Date of publication of application: **14.01.2026**
(21) Application number: 24306181.9
(22) Date of filing: 12.07.2024
(51) Int. Cl.: A61P 9/00, A61K 31/4184, A61K 31/44, A61K 31/496, A61P 25/00, A61P 29/00, A61P 37/00, C07D 401/04

(54) **ANTAGONIST HETEROCYCLIC COMPOUNDS INVOLVED IN THE CGAS-STING ROUTE**

(71) Applicant: Centre National de la Recherche Scientifique, 75016 Paris (FR); Université d'Orléans, 45100 Orléans (FR)
(72) Inventor: AGROFOGLIO, Luigi, 45100 Orléans (FR); ROY, Vincent, 45480 Outarville (FR); MAGAND, Jérémy, 42500 Le Chambon-Feugerolles (FR); QUESNIAUX, Valérie, 45560 Saint Denis en Val (FR)
(74) Representative: Novagraaf Technologies

(57) **Abstract**

Compound of formula (I): 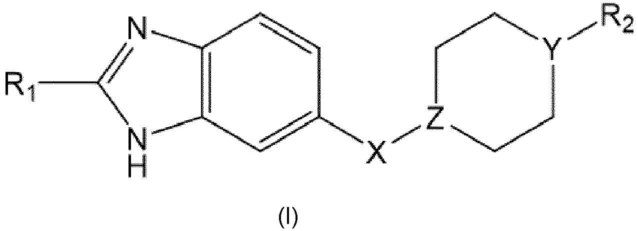 or their pharmaceutically acceptable salts, hydrates, solvates, esters or their optical isomers, racemates, diastereoisomers, enantiomers, tautomers, or a mixture thereof;
for use in the treatment of at least one disorder selected among cardiovascular disorders, neurological disorders, autoimmune and inflammatory diseases.

## Description

### FIELD OF THE INVENTION

The present invention relates to compounds comprising a benzimidazole scaffold, and the use of such compounds for the treatment of diseases. The invention also relates to pharmaceutical compositions comprising said compounds as active ingredient. In particular the compounds of the invention comprising a benzimidazole scaffold are used for the treatment of cardiovascular disorders, neurological disorders, autoimmune and inflammatory diseases.

### BACKGROUND OF THE INVENTION

The STING protein is involved in the innate antimicrobial immune defence system, and beyond, in sterile inflammation. Upon sensing cytosolic dsDNA the enzyme cGAS produces the cyclic di-nucleotide 2'3' cyclic GMP-AMP (cGAMP) which binds and activates STING, the STING protein then further activates the immune response cascade by stimulating interferon genes. The STING protein activates a set of genes that produce signalling molecules (cytokines) that help the body's defence cells to fight microbes. However, deregulation of this pathway, particularly when STING is over-expressed in the absence of pathogens, can be the cause of a wide range of diseases including autoimmune, inflammatory, cardiovascular and neurological disorders.

The treatment of autoimmune or chronic inflammatory diseases remains an unmet medical need due to their complex pathogenesis and critical therapeutic requirements. Growing evidence indicates that modulation of the innate immune system by targeting STING is an emerging strategy for combating autoimmune diseases.

Compared with STING agonists, the development of STING inhibitors is still in its infancy and no candidate has yet entered clinical investigation. It is therefore extremely important to identify a selective and effective STING inhibitor for clinical evaluation in order to provide proof of concept for this approach.

The goal of the present invention is to provide a set of compounds exhibiting antagonistic activity toward the cGAS-STING pathway, comparable or even superior to the reference products H-151 and SN-011. Besides, those compounds may also exhibit inhibitory activity on the production of type I and/or III interferons and Interferon-stimulated genes (ISG) molecules such as CXCL10.

The inventors have studied compounds among those described for the treatment of Ebola virus or HIV virus, in the application EP 3 296 297 A1, and have determined whether they could also exhibit antagonistic activity toward the cGAS-STING pathway.

### BRIEF DESCRIPTION OF THE DRAWINGS

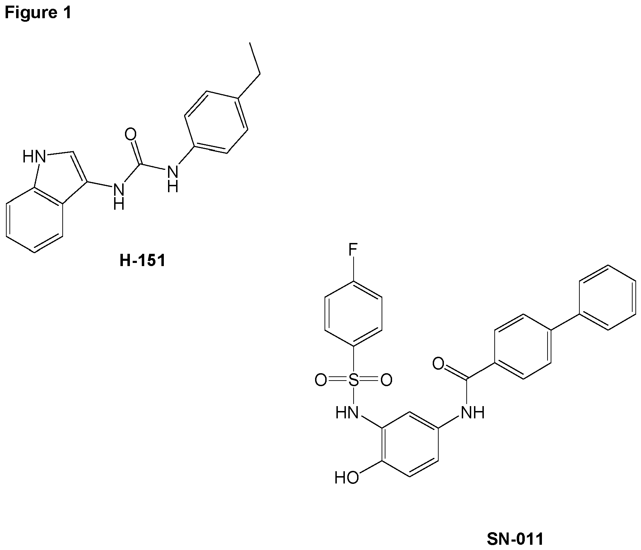

**Figure 1** represents the chemical structure of compounds H-151 and SN011;

### DEFINITIONS

As used hereabove or hereafter:
"Alkyl" means an aliphatic hydrocarbon group which may be straight or branched having 1 to 6 carbon atoms in the chain. "Branched" means that one or more lower alkyl groups such as methyl, ethyl or propyl are attached to a linear alkyl chain. Exemplary alkyl groups include methyl, ethyl, n-propyl, i-propyl, n-butyl, t-butyl, n-pentyl, 3-pentyl.

"Alkoxyl" means an O-alkyl group, the alkyl group being as described above.

"Alken" or "alkenyl" means an aliphatic hydrocarbon group containing a carbon-carbon double bond and which may be straight or branched having 2 to 6 carbon atoms in the chain. Preferred alkenyl groups have 2 to 6 carbon atoms in the chain; and more preferably about 2 to 4 carbon atoms in the chain. Exemplary alkenyl groups include ethenyl, propenyl, *n*-butenyl, *i*-butenyl, 3-methylbut-2-enyl, *n*-pentenyl.

"Aryl" means an aromatic monocyclic or multicyclic hydrocarbon ring system of 6 to 14 carbon atoms, preferably of 6 to 10 carbon atoms, substituted or not. Exemplary aryl groups include phenyl or naphthyl.

"Halogen atom" refers to fluorine, chlorine, bromine or iodine atom; preferably fluorine atom.

"Haloalkyl" refers to an alkyl group as described above substituted by at least one halogen atom as described above, for example substituted by 1 to 6 halogen atoms, preferably by 1 to 3 halogen atoms. Exemplary haloalkyl group includes trifluoromethyl group.

"Haloalkoxyl" refers to an alkoxyl group as described above substituted by at least one halogen atom as described above, for example substituted by 1 to 6 halogen atoms, preferably by 1 to 3 halogen atoms, for example -OCF₃.

As used herein, the term "heteroaryl" refers to a 5 to 14, preferably 5 to 10-membered aromatic hetero, mono-, bi- or multicyclic ring comprising at least one heteroatom preferably chosen among: O, N or S, preferably from 1 to 5 heteroatoms, for example from 1 to 4 heteroatoms, expecially one, two or three heteroatoms. Examples include pyrrolyl, pyridyl, pyrazolyl, thienyl, pyrimidinyl, pyrazinyl, tetrazolyl, indolyl, quinolinyl, purinyl, imidazolyl, thienyl, thiazolyl, benzothiazolyl, furanyl, benzofuranyl, 1,2,4-thiadiazolyl, isothiazolyl, triazoyl, tetrazolyl, isoquinolyl, benzothienyl, isobenzofuryl, pyrazolyl, carbazolyl, benzimidazolyl, isoxazolyl, pyridyl-N-oxide, as well as the fused systems resulting from the condensation with a phenyl group.

"Alkyl", "aryl", "alkoxyl", "haloalkoxyl", "haloalkyl", "heteroaryl" and the likes refers also to the corresponding "alkylene", "arylene", "alkoxylene", "haloalkoxylene", "haloalkylene" "heteroarylene" and the likes which are formed by the removal of two hydrogen atoms. Alkyl and alkylene are used herein interchangeably.

As used herein, the expression "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein, "pharmaceutically acceptable salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like, including mono, di or tri-salts thereof; and the salts prepared from organic acids such as acetic, propionic, succinic, tartaric, citric, methanesulfonic, benzenesulfonic, glucoronic, glutamic, benzoic, salicylic, toluenesulfonic, oxalic, fumaric, maleic, lactic and the like. Further addition salts include ammonium salts such as tromethamine, meglumine, epolamine, etc., metal salts such as sodium, potassium, calcium, zinc or magnesium.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 20th ed., Mack Publishing Company, Easton, PA, 2000, the disclosure of which is hereby incorporated by reference.

The compounds that will be further presented in the context of the invention having geometrical and stereoisomers are also part of the invention.

The compounds of the present invention may be prepared in a number of ways well-known to those skilled in the art. The compounds can be synthesized, for example, by application or adaptation of the methods known by the skilled person. The appropriate modifications and substitutions will be readily apparent and well known or readily obtainable from the scientific literature to those skilled in the art.

It will be appreciated that the compounds of the present invention may contain one or more asymmetrically substituted carbon atoms, and may be isolated in optically active or racemic forms. Thus, all chiral, diastereomeric, racemic forms, isomeric forms of a structure are covered, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes as shown in the examples, which can be adapted by the skilled person based on its knowledge with regard to the desired compounds.

The present invention also relates to pharmaceutical compositions comprising at least one compound as active principle. Preferably, the present invention also relates to pharmaceutical compositions comprising at least one compound as active principle and at least one excipient pharmaceutically acceptable.

According to the invention, the terms "patient" or "patient in need thereof', are intended for an animal such as a valuable animal for breeding, company or preservation purposes, or a human or a human child, being affected or likely to be affected with one or more disesases and conditions described herein. Preferably, the patient is human.

The identification of those subjects who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those subjects who are in need of such treatment.

As used herein, a "therapeutically effective amount" refers to an amount of a compound of the present invention which is effective in preventing, reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of a compound of formula (I), which is required to achieve the desired biological effect, will vary depending upon a number of factors, including the chemical characteristics of the compounds employed, the potency of the compounds, the type of disease, the species to which the patient belongs, the diseased state of the patient, the route of administration, the bioavailability of the compound by the chosen route, all factors which dictate the required dose amounts, delivery and regimen to be administered.

As used herein, "pharmaceutically acceptable excipient" includes any carriers, diluents, adjuvants, or vehicles, such as preserving or antioxidant agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well-known in the art. Except insofar as any conventional media or agent is incompatible with the active ingredient, its use in the therapeutic compositions is contemplated. Supplementary active ingredients can also be incorporated into the compositions as suitable therapeutic combinations.

In the context of the invention, the term "treating" or "treatment", as used herein, means reversing, alleviating, inhibiting the progress of, or preventing the disorder or condition to which such term applies, or one or more symptoms of such disorder or condition.

The preferred dosage of drug to be administered is likely to depend on such variables as the type and extent of progression of the disease or disorder, the overall health status of the particular patient, the relative biological efficacy of the compound selected, the formulation of the compound, the route of administration (intravenous, intramuscular, or other), the pharmacokinetic properties of the compound by the chosen delivery route, and the speed (bolus or continuous infusion) and schedule of administrations (number of repetitions in a given period of time).

The compound of the present invention is also capable of being administered in unit dose forms, wherein the expression "unit dose" means a single dose which is capable of being administered to a patient, and which can be readily handled and packaged, remaining as a physically and chemically stable unit dose comprising either the active compound itself, or as a pharmaceutically acceptable composition, as described hereinafter. Compounds provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasally, particularly in the form of powders, nasal drops, or aerosols; or dermally, for example, topically in ointments, creams, lotions, gels or sprays, or via transdermal patches.

The compositions may conveniently be administered in unit dosage form and may be prepared by any of the methods well-known in the pharmaceutical art.

For oral administration, tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolved, modified-release or sustained-release preparations and formulations, and wherein such sustained-release formulations are preferably bi-modal. Preferred tablets contain lactose, cornstarch, magnesium silicate, croscarmellose sodium, povidone, magnesium stearate, or talc in any combination.

Liquid preparations for parenteral administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.

Alternative modes of administration include formulations for airway administration or inhalation, which include such means as dry powder, aerosol, spray or drops. They may be aqueous solutions or microdroplets containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

In the present invention "compound X for the treatment of Y" is equivalent to "compound X for use in a method for the treatment of Y" or "compound X for use in the therapy of Y".

The present invention also relates to the use of at least one compound of the invention or of the composition of the invention for the preparation of a medicine for the treatment of diseases.

The present invention also relates to a method of treatment of viral diseases comprising the administration of a therapeutically effective amount of at least one compound of the invention or of the composition of the invention to a patient in need thereof.

### BRIEF DESCRIPTION OF THE INVENTION

The present invention relates to a compound having the following formula (I): 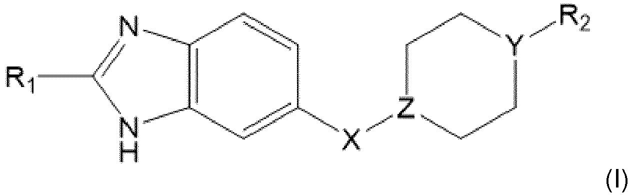 in which:
▪ X represents:
   ∘ a C=O group;
   ∘ an alkyl group, linear or branched, comprising 1 to 6 carbon atoms ; or
   ∘ a group of formula -NR-(C₁-C₆)alkyl-, the alkyl being linear or branched;
▪ Y and Z represents independently a CH or a nitrogen atom;
▪ R₁ represents:
   ∘ an aryl group, comprising 6 to 10 carbon atoms, non-substituted or substituted by at least one group chosen among: haloalkyl, haloalkoxyl, alkyl or alkoxyl group, the alkyl being linear or branched, and comprising 1 to 6 carbon atoms;
   ∘ an heteroaryl group, comprising 6 to 10 members, non-substituted or subsituted by at least one group chosen among: haloalkyl, haloalkoxyl, alkyl or alkoxyl group, the alkyl being linear or branched, and comprising 1 to 6 carbon atoms;
   ∘ a CH-(aryle)₂ group, the aryl comprising 6 to 10 carbon atoms, non-substituted or subsituted by at least one group chosen among: haloalkyl, haloalkoxyl, alkyl or alkoxyl group, the alkyl being linear or branched, and comprising 1 to 6 carbon atoms; or
   ∘ a CH-(heteroaryl)₂ group, the heteroaryl comprising 6 to 10 members, non-substituted or subsituted by at least one group chosen among: haloalkyl, haloalkoxyl, alkyl or alkoxyl group, the alkyl being linear or branched, and comprising 1 to 6 carbon atoms;
▪ R₂ represents :
   ∘ a (C₁-C₆)alkyl-aryl-R₃ group, the aryl comprising 6 to 10 carbon atoms and the alkyl being linear or branched;
   ∘ a C(O)N(R)-aryl-R₅ group, the aryl comprising between 6 to 10 carbon atoms;
   ∘ a C(O)-(C₁-C₆)alkyl-O-aryl-Hal group, with Hal represents a halogen, the aryl comprising between 6 and 10 carbon atoms and the alkyl being linear or branched;
   ∘ a C(O)O-(C₁-C₆)alkyl-aryl group, the aryl comprising between 6 to 10 carbon atoms and the alkyl being linear or branched;
   ∘ a O-aryl-R₃ group, the aryl comprising between 6 to 10 carbon atoms;
   ∘ a C(O)N(R)-(C₁-C₆)alkyl group, the alkyl being preferably substituted by a -C(=O)O-R' function; or
   ∘ a C(O)N(R)-(C₁-C₆)alkyl-aryl group, the aryl comprising between 6 to 10 carbon atoms;
▪ R₃ represents a OR₄ group, haloalkyl or haloakoxyl, wherein the alkyl is linear or branched, and comprises 1 to 6 carbon atoms;
▪ R₄ represents a (C₁-C₆)alkyl-aryl-COOR group, a haloalkyl group or a (C₁-C₆)alkylaryl-(C₁-C₆)alkyl-PO(OR)₂ group, wherein alkyl is linear or branched and comprises 1 to 6 carbon atoms and the aryl comprises between 6 and 10 carbon atoms;
▪ R₅ represents an alkyl group, linear or branched, comprising 1 to 6 carbon atoms; or a haloalkyl, linear or branched, comprising 1 to 6 carbon atoms;
▪ R or R' represents independently an alkyl group, linear or branched, comprising 1 to 6 carbon atoms, or a hydrogen atom;
   or their pharmaceutically acceptable salts, hydrates, solvates, esters or their optical isomers, racemates, diastereoisomers, enantiomers, tautomers, or a mixture thereof;
   for use in the treatment of at least one disorder selected among cardiovascular disorders, neurological disorders, autoimmune and inflammatory diseases.

Preferably, in the compound of formula (I), R₁ is chosen among:
▪ a phenyl group, non-substituted or substituted by at least one group chosen among: haloalkyl, haloalkoxyl, alkyl or alkoxyl group, the alkyl being linear or branched, and comprising 1 to 6 carbon atoms;
▪ a pyridine group non-substituted or substituted by at least one group chosen among: haloalkyl, haloalkoxyl, alkyl or alkoxyl group, the alkyl being linear or branched, and comprising 1 to 6 carbon atoms;
▪ a pyrimidine group, non-substituted or substituted by at least one group chosen among: haloalkyl, haloalkoxyl, alkyl or alkoxyl group, the alkyl being linear or branched, and comprising 1 to 6 carbon atoms; or
▪ a benzhydryl group non-substituted or substituted by at least one group chosen among: haloalkyl, haloalkoxyl, alkyl or alkoxyl group, the alkyl being linear or branched, and comprising 1 to 6 carbon atoms.

Preferably, in the compound of formula (I), R₁ is chosen among:
▪ a phenyl group non-substituted or substituted by at least one group chosen among: fluoroalkyl, fluoroalkoxyl, alkyl or alcoxyl group, the alkyl being linear or branched, and comprising 1 to 6 carbon atoms;
▪ a pyridine group non-substituted or substituted by at least one group chosen among: haloalkyl, haloalkoxyl, alkyl or alkoxyl group, the alkyl being linear or branched, and comprising 1 to 6 carbon atoms; or
▪ a benzhydryl group non-substituted or substituted by at least one group chosen among: fluoroalkyl, fluoroalkoxyl, alkyl or alcoxyl group, the alkyl being linear or branched, and comprising 1 to 6 carbon atoms.

Preferably, in the compound of formula (I), X represents:
∘ a (C=O) group;
∘ an alkyl group, linear or branched, comprising 1 to 3 carbon atoms; or
∘ a group of formula -NH-(C₁-C₃)alkyl-, the alkyl being linear or branched.

Preferably, in the compound of formula (I), Z represents a CH group and Y represents a nitrogen atom.

Preferably, in the compound of formula (I), Y and Z represents a nitrogen atom.

Preferably, said compound (I) is selected among one of the followings:

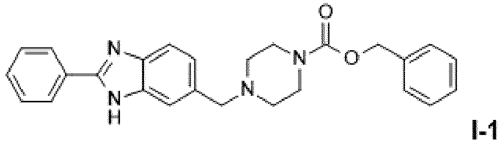

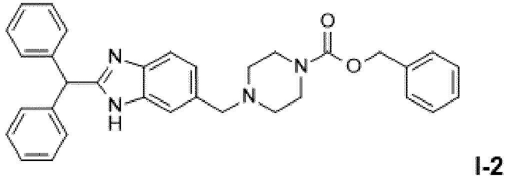

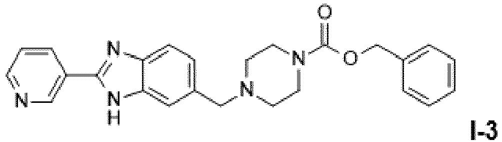

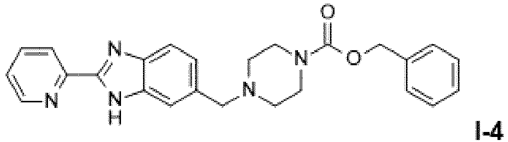

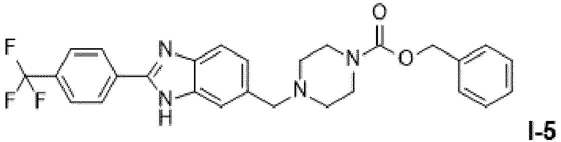

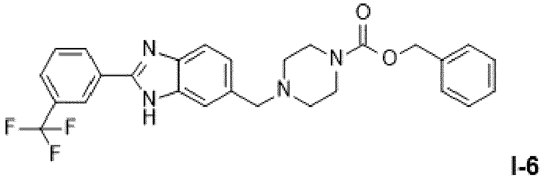

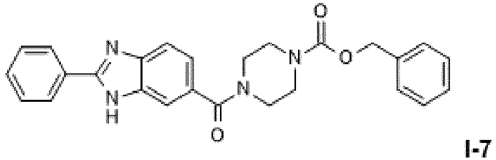

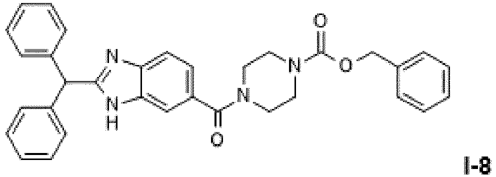

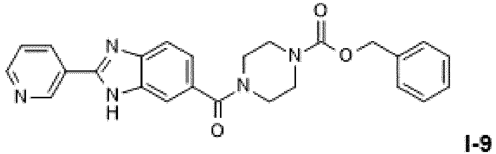

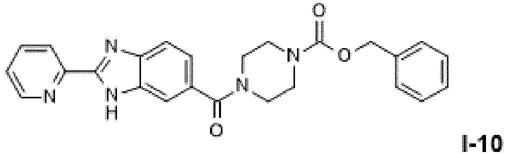

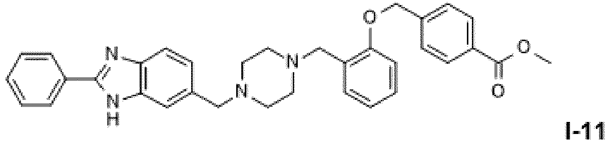

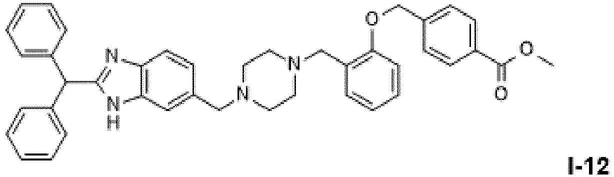

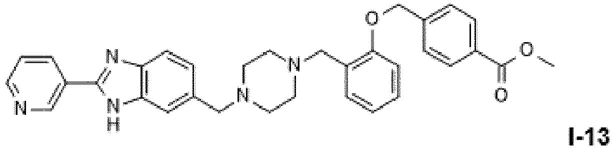

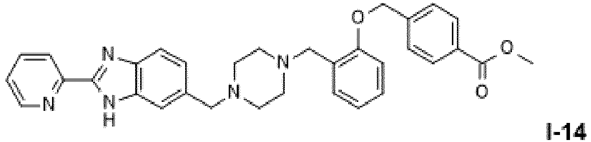

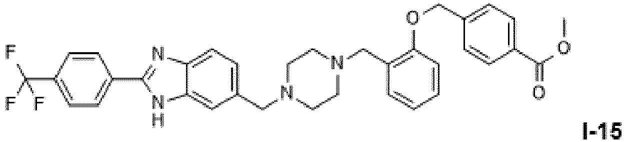

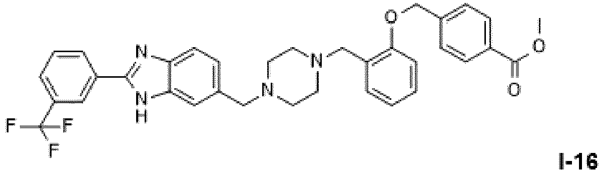

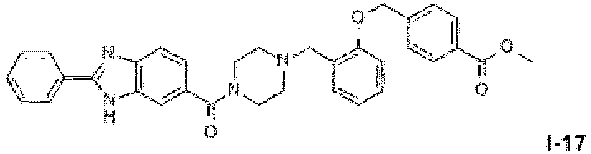

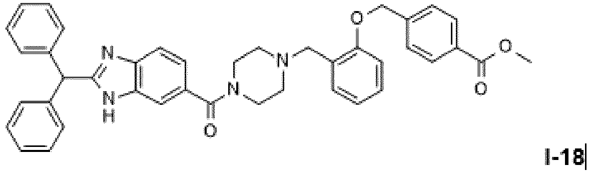

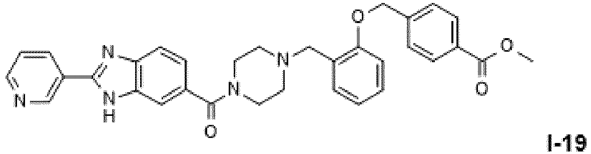

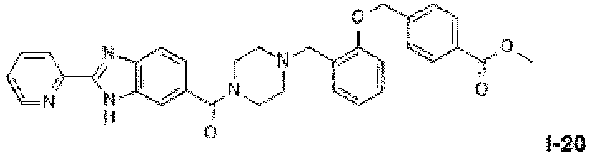

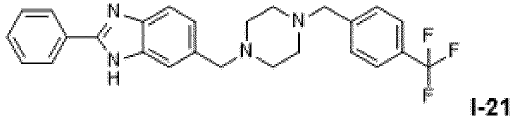

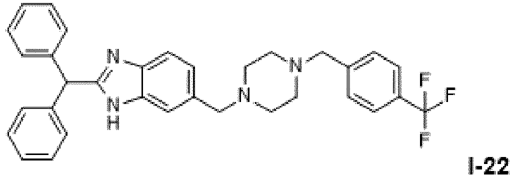

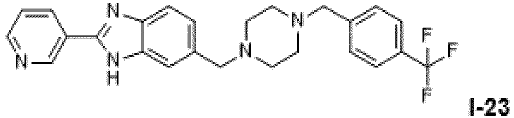

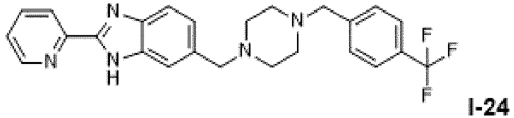

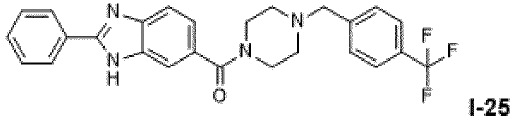

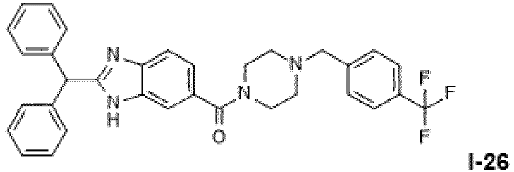

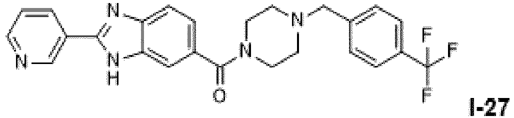

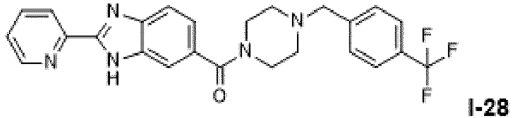

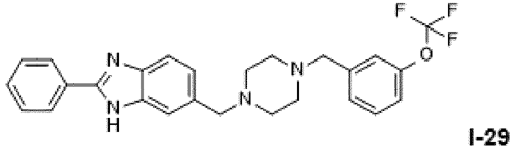

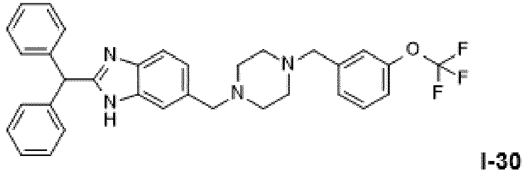

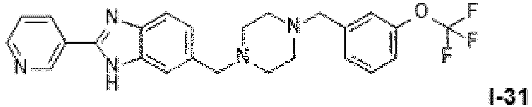

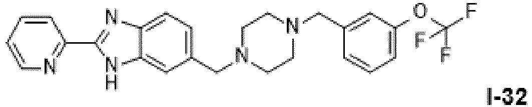

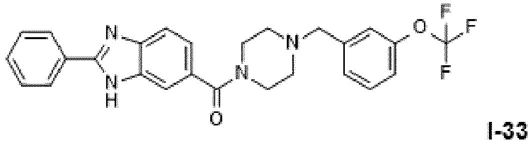

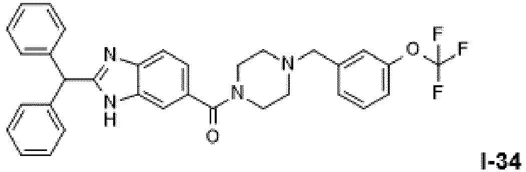

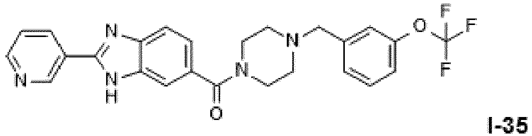

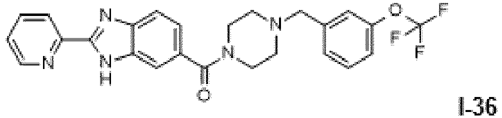

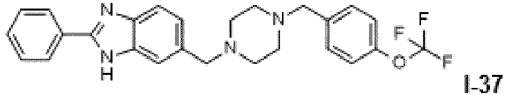

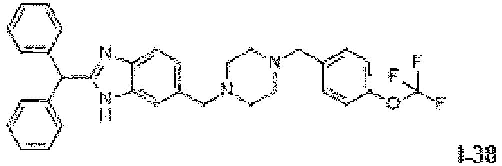

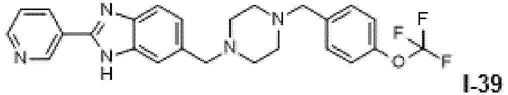

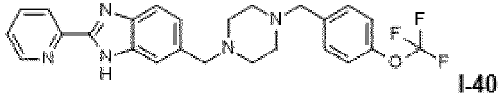

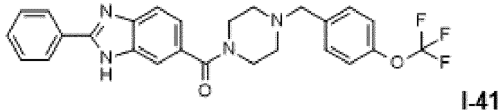

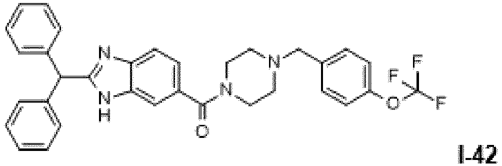

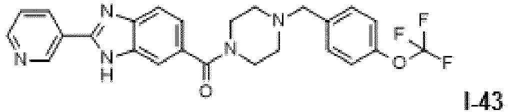

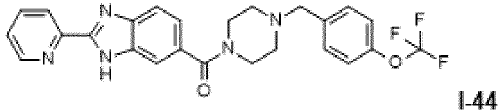

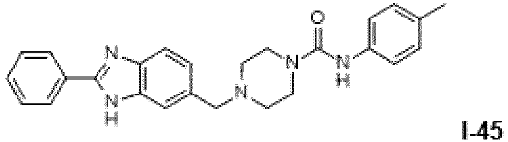

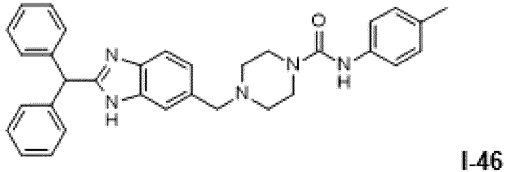

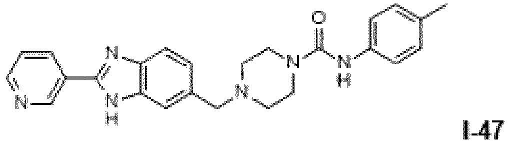

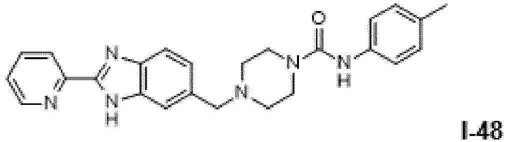

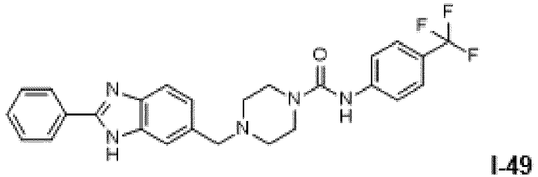

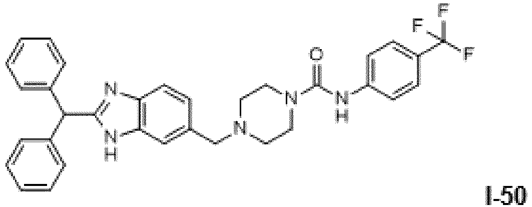

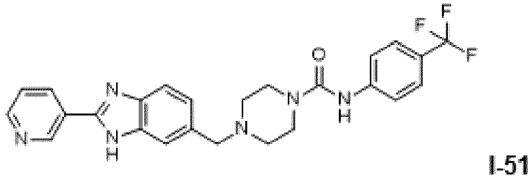

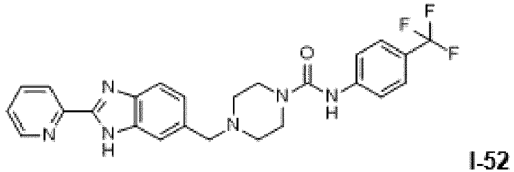

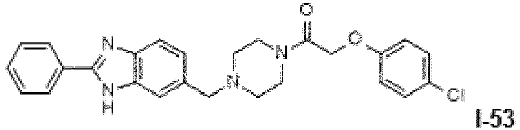

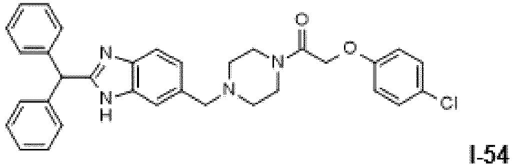

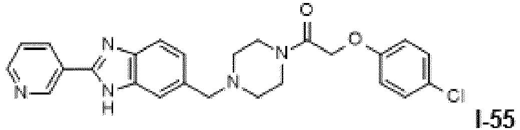

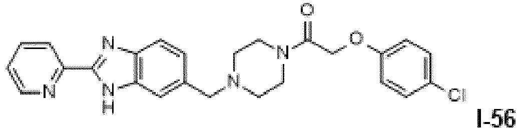

The present invention relates also to a compound of formula (I) as precedently described in the context of the present invention, for the treatment of at least one of the autoimmune and inflammatory diseases selected among : interferonopathies such as the so-called Aicardi-Goutières-Syndrome (AGS), or the lupus-like disease such as STING-associated vasculopathy with onset in infancy (SAVI), Familial chilblain lupus; chronic diseases such as lupus, including subtypes of systemic lupus erythematosus (SLE), lupus nephritis (LN), and dermatomyositis, or Sjogren's Syndrome (SS); rheumatoid arthritis; inflammatory bowel disease (IBD) such as ulcerative colitis or Crohn's disease; sepsis; lung inflammatory diseases such as Acute Respiratory Distress Syndrome (ARDS), silicosis, chronic obstructive pulmonary disease (COPD); Metabolic diseases such as Nonalcoholic steatohepatitis (NASH), Alcoholoc liver disease, cirrhosis; Acute pancreatitis; nephropathies, chemo-induced acute kidney injury; chonic inflammation from different origins including viral infection; cancer including Colorectal cancer, Skin cancer, metastasis; senescence and aging-associated inflammation.

The present invention relates also to a compound of formula (I) for the treatment of at least one of the cardiovascular or neurological disorders, selected among : Myocardial infarction, Chronic heart failure, Endomyocardial fibrosis; Neuroinflammatory diseases such as Parkinson's disease, Alzheimer's disease, Amyotrophic Lateral Sclerosis (ALS), or Age-dependent macular degeneration.

Preferably, the compound (I) is for the treatment of autoimmune diseases involving the cGAS/STING pathway and interferon, preferably type I or III interferons.

The present invention relates also to Compound of formula (II) : 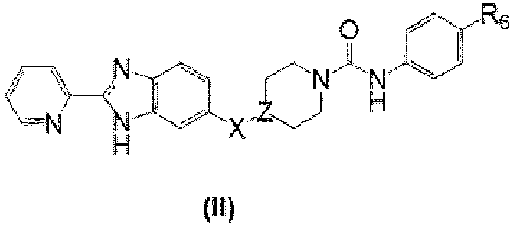
R₆ represents one of the following group selected among:
   - a (C₁-C₃)alkyl group preferably substituted by a -C(=O)O-R' function;
   - an aryl comprising 6 carbon atoms, said aryl being preferably substituted by a group selected among one of the followings: -CF₃, Cl, NO₂, NH₂, and methyl; and
   - a (C₁-C₆)alkyl-aryl group, the alkyl group being preferably -CH₂- and the aryl comprising 6 carbon atoms;
X represents an alkyl group, linear or branched, comprising 1 to 3 carbon atoms; or a group of formula -NH-(C₁-C₃)alkyl-, the alkyl being linear or branched; and
Z represents a CH or a nitrogen atom;
with the exclusion of the following compounds: 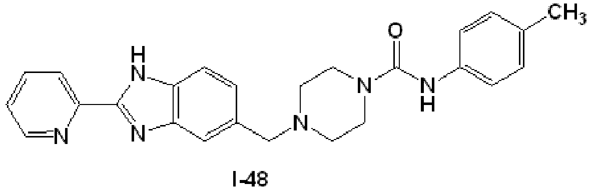 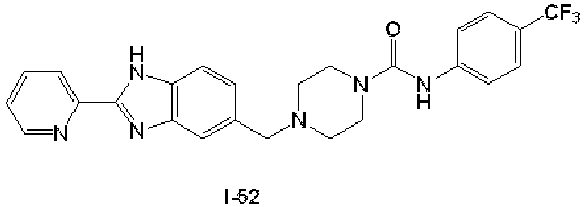
or their pharmaceutically acceptable salts, hydrates, solvates, esters or their optical isomers, racemates, diastereoisomers, enantiomers, tautomers, or a mixture thereof.

Preferably, the compound according to formula (II) is selected among the followings:

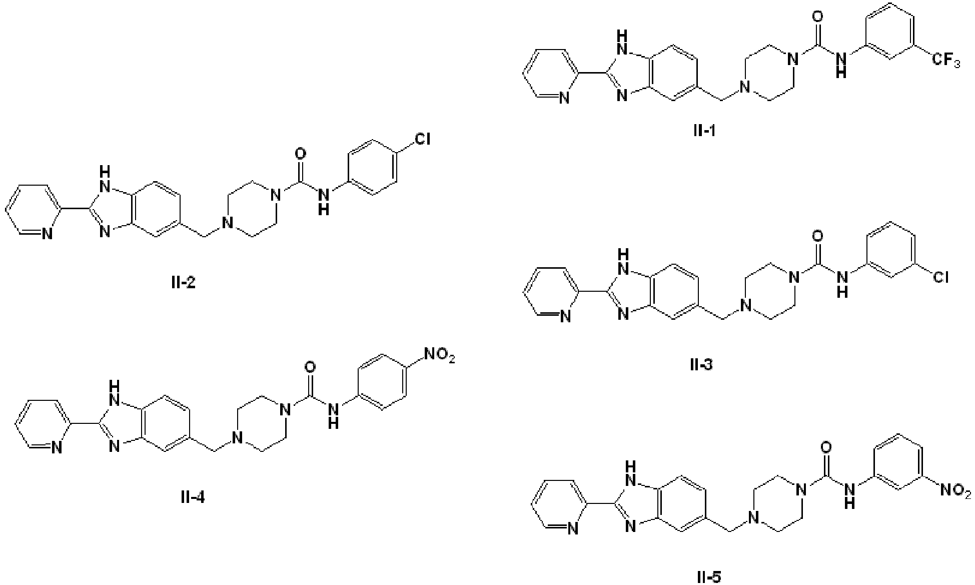

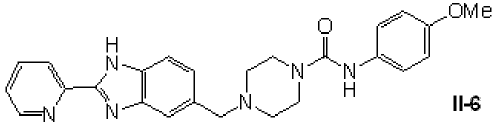

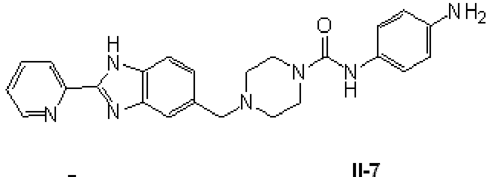

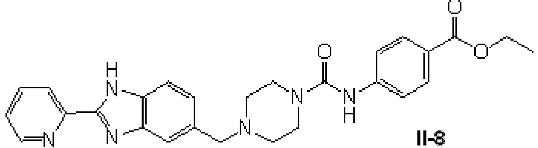

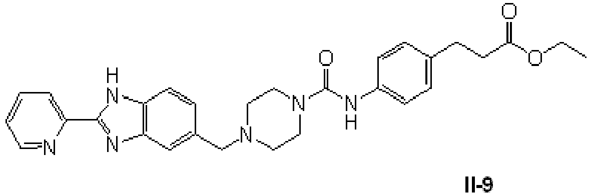

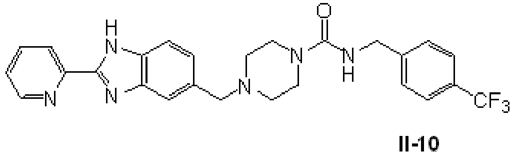

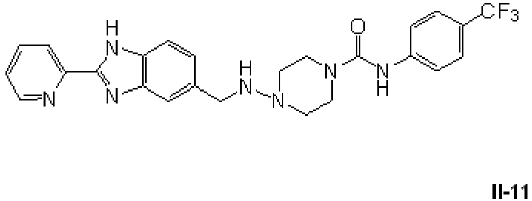

Preferably, the compound according to:
- anyone of the formula according to formula (II), with the exception of (I-48) or (I-52), or
- the compound selected among one of the formulas according to anyone among formula (II-1) to (II-11) as precedenlty presented, for use as a medicament. Preferably said compound is for use in the treatment of at least one disorder selected among cardiovascular disorders, neurological disorders, autoimmune and inflammatory diseases; and more preferably for use in the treatment of at least one of the precedently presented disease or disorder in the context of the present invention.

The present invention also relates to a pharmaceutical composition comprising at least one compound according to:
- anyone of the formula according to formula (II), with the exception of (I-48) or (I-52), or
- the compound selected among one of the formulas according to anyone among formula (II-1) to (II-11) as precedenlty presented, as active principle, and optionally a pharmaceutically acceptable excipient.

### DETAILED DESCRIPTION OF CERTAIN EXEMPLARY EMBODIMENTS OF THE INVENTION

### Part I. Purification and chemical study of the molecules

### Material and methods:

Commercially available chemicals were provided as reagent grade and used as received. Some reactions requiring anhydrous conditions were carried out using oven-dried glassware and under an atmosphere of dry Argon. All anhydrous solvents were provided from commercially sources as very dry reagents. The reactions were monitored by thin layer chromatography (TLC) analysis using silica gel precoated plates (Kieselgel 60F254, E. Merck). Compounds were visualized by UV irradiation and/or spraying with sulfuric acid (H₂SO₄ 5% in ethanol) stain followed by charring at average 150 °C. Flash column chromatography was performed on Silica Gel 60 M (0.040-0.063 mm, E. Merck). The infrared spectra were measured with Perkin-Elmer Spectrometer. The ¹H and ¹³C NMR spectra were recorded on BrukerAvance DPX 250 or BrukerAvance 400 Spectrometers. Chemical shifts are given in ppm and are referenced to the deuterated solvent signal or to TMS as internal standard and multiplicities are reported as s (singlet), d (doublet), t (triplet), q (quartet) and m (multiplet). Carbon multiplicities were assigned by distortion less enhancement by polarization transfer (DEPT) experiments. ¹H and ¹³C signals were attributed on the basis of H-H and H-C correlations. High Resolution Mass spectra were performed on a Bruker Q-TOF MaXis spectrometer by the "Fédération de Recherche" ICOA/CBM (FR2708) platform. LC-MS data was acquired on a Thermo-Fisher UHPLC-MSQ system equipped with an electron spray ionization source (ESI). The temperature of the source was maintained at 350 °C. Initially, the cone voltage was set at 35 V and after 5 min was increased to 75V. In full scan mode, data was acquired between 100 and 1000 m/z in the positive mode with a 1.00 S scan time. In addition, a UV detection was performed with a Diode array detector at three wavelengths 273, 254 and 290 nm, respectively. A water/methanol (70%/30%) solution mixture with 0.1% formic acid was used as mobile phase. The composition of the mobile phase was increased to 100% methanol with 0.1% formic acid with a 7% ramp. The flow rate was set at 0.300 mL.min-1. Samples diluted in the mobile phase were injected (3 µL) on a C18 column (X-terra, Waters), 2.1 mm internal diameter, and 100 mm length placed into an oven at 40 °C. Electronic extraction of ions was performed and the subsequent areas under the corresponding chromatographic peaks determined.

### Part II. Syntheses and Characteristization

### General procedure (A) for the synthesis of piperazine moieties:

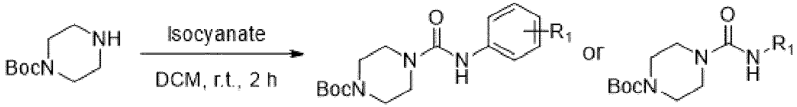

To a solution of Boc-piperazine (1.0 eq.) in CH₂Cl₂ (12 mL) was added isocyanate (1.1 eq.). The reaction mixture was stirred, under argon atmosphere, at room temperature for 2 hours. The resulting mixture was diluted with CH₂Cl₂, the organic layer was washed with water and brine, dried over Na₂SO₄, and concentrated *in vacuo.* The crude was purified by flash-column chromatography (silica gel, CH₂Cl₂ 100% -> CH₂Cl₂/MeOH, 98:2) to obtain the desired product.

### General procedure (B) for Boc- deprotection :

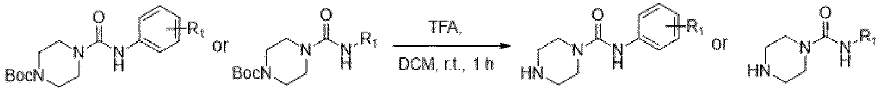

To a solution of the Boc-piperazine derivative (1.0 eq.) in CH₂Cl₂ was added then TFA (16.0 eq.). The reaction mixture was stirred for 1 to 2 hours at room temperature. The resulting mixture was coevaporated with toluene and concentrated under reduced pressure. The residue was then dissolved in EtOAc and sat. aq. NaHCO₃ was added. The aqueous layer was extracted with EtOAc and the combined organic layers were collected, dried over Na₂SO₄, and concentrated under reduced pressure. The crude residue was purified by flash-column chromatography (silica gel, CH₂Cl₂/MeOH, 95:5) to give the desired product.

### General procedure (C) for the coupling reaction :

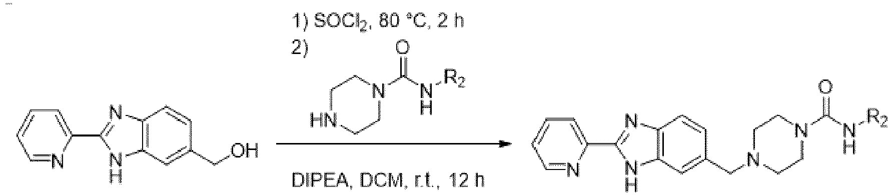

To the flask with hydroxymethyl benzimidazole derivative (200 mg, 0.89 mmol, 1.0 eq.) was added SOCl₂ (1.9 mL, 26 mmol, 29.0 eq.) and the resulting mixture was stirred at 80 °C for 2 hours. After cooling to room temperature, the SOCl₂ was evaporated and the obtained residue was diluted with CH₂Cl₂ (15 mL) and cooled to 0 °C. Then, DIPEA (0.9 mL, 5.34 mmol, 6.0 eq.) was added to the reaction mixture, followed by the piperazine derivative (1.0 eq.). The ice bath was removed, and the reaction mixture was stirred for 12 hours at room temperature. Water was added, and the aqueous layer was extracted 4 times with CH₂Cl₂. Combined organic layers were rinsed with brine, dried over Na₂SO₄ and concentrated *in vacuo.* The crude residue was purified by flash-column chromatography (silica gel, CH₂Cl₂/MeOH) to provide the desired product.

### 2-(pyridin-2-yl)-1H-benzo[d]imidazole-5-carboxylic acid (1)

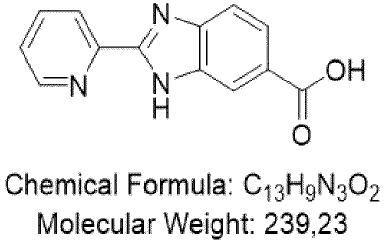

To a solution of 3,4-diaminobenzoic acid (2.0 g, 13.1 mmol, 1.0 eq.) in acetic acid (30 mL) was slowly added picolinaldehyde (1.25 mL, 13.1 mmol, 1.0 eq.), and the reaction mixture was stirred at 120 °C for 5 hours. The reaction mixture was cooled down to room temperature and half of the solvent was then evaporated under reduced pressure. The obtained solid was filtered, washed with water and dried under *vacuum* to give **1** (2.5 g, 10.5 mmol) as a brown solid in 80% yield. The crude residue was directly used in the next step without further purification. ¹H NMR (250 MHz, MeOD): δ 8.69 (ddd, *⁵J* = 1.0 Hz, *⁴J* = 1.8 Hz, *⁵J* = 4.8 Hz, 1H, H₁₄), 8.32 (dd, *⁵J* = 0.7 Hz, *⁴J* = 1.6 Hz, 1H, H₄), 8.24 (dt, *⁴J* = 1.1 Hz, *³J* = 8.0 Hz, 1H, H₁₁), 7.98 - 7.86 (m, 2H, H₆, H₁₂), 7.62 (dd, *⁴J* = 0.7 Hz, *³J* = 8.6 Hz, 1H, H₇), 7.48 - 7.33 (m, 1H, H₁₃). ¹³C NMR (63 MHz, MeOD) δ 174.4 (quat C, COOH), 152.4 (quat C, C₂), 149.5 (CH, C₁₄), 148.1 (quat C, C₁₀), 140.3 (quat C, C₈), 137.1 (CH, C₁₂), 133.3 (quat C, C₉), 124.6 (2x CH, C₆, C₁₃), 124.5 (quat C, C₅),121.2 (CH, C₁₁), 116.5 (CH, C₄), 113.9 (CH, C₇). LC-MS (ESI⁺) *m*/*z* = 240.1 [M+H]⁺. The spectroscopic data are in accordance with those reported in literature (CAS # 669070-64-8).

### Methyl 2-(pyridin-2-yl)-1H-benzo[d]imidazole-5-carboxylate (2)

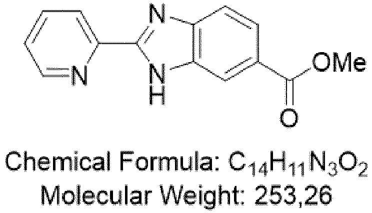

To a solution of compound **1** (1.2 g, 5.02 mmol, 1.0 eq.) in MeOH (12 mL) cooled at 0°C, was slowly added SOCl₂ (0.8 mL, 11.0 mmol, 2.2 eq.). The reaction mixture was heated at 80 °C for 4 hours. After cooling at room temperature, water was added, and the mixture was concentrated under reduced pressure. The pH was adjusted to 7 with a sat. aq. The aqueous phase was extracted three times with EtOAc. The combined organic layers were dried over Na₂SO₄ and concentrated *in vacuo.* The crude residue was purified by flash-column chromatography (silica gel, EtOAc, 100%) to give **(2)** (1.14 g, 4.50 mmol) as a beige solid in 95% yield. **Rf=** 0,46 (silica, PE/EtOAc, 9 :1). **Mp=** 192 ± 2 °C. **IR (cm⁻¹):** 3319, 1696, 1436, 1401, 1324, 1276, 1214, 1114. **¹H NMR (250 MHz, MeOD):** δ 8.69 (ddd, *⁵J* = 1.0 Hz, *⁴J* = 1.8 Hz, *⁵J* = 4.8 Hz, 1H, H₁₄), 8.32 (dd, *⁵J* = 0.7 Hz, *⁴J* = 1.6 Hz, 1H, H₄), 8.24 (dt, *⁴J* = 1.1 Hz, *³J* = 8.0 Hz, 1H, H₁₁), 7.98 - 7.86 (m, 2H, H₆, H₁₂), 7.62 (dd, *⁴J* = 0.7 Hz, *³J* = 8.6 Hz, 1H, H₇), 7.48 - 7.33 (m, 1H, H₁₃), 3.91 (s, 3H, CH₃). **¹³C NMR (63 MHz, MeOD):** δ 174.4 (quat C, COOH), 152.4 (quat C, C₂), 149.5 (CH, C₁₄), 148.1 (quat C, C₁₀), 140.3 (quat C, C₈), 137.1 (CH, C₁₂), 133.3 (quat C, C₉), 124.6 (2x CH, C₆, C₁₃), 124.5 (quat C, C₅), 121.2 (CH, C₁₁), 116.5 (CH, C₄), 113.9 (CH, C₇), 52.5 (CH₃). **LC-MS (ESI⁺)** *m*/*z* = 254.2 [M+H]⁺. The spectroscopic data are in accordance with those reported in literature (CAS # 864274-78-2).

### (2-(pyridin-2-yl)-1H-benzo[d]imidazol-5-yl)methanol (3)

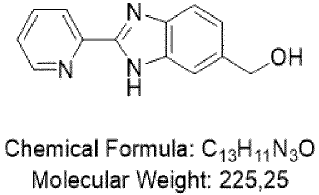

To a solution of compound **2** (2.0 g, 7.90 mmol, 1.0 eq.) in dry THF (100 mL) cooled at 0 °C, was slowly added LiAIH4 (600 mg, 15.8 mmol, 2.0 eq.) under argon atmosphere. The ice bath was removed, and the reaction was stirred for 3 hours at 50 °C. The reaction mixture was cooled at 0 °C and then quenched with sat. aq. NaHCO₃. The mixture was concentrated under reduced pressure and the resulting aqueous phase was extracted four times with EtOAc. The combined organic layers were dried over Na₂SO₄ and concentrated *in vacuo.* The crude residue was purified by flash-column chromatography (silica gel, CH₂Cl₂/MeOH, 95:5) to give **3** (1.5 g, 6.71 mmol) as a yellow solid in 85% yield. **Rf=** 0,32 (CH₂Cl₂/MeOH 9 :1). **Mp=** 170 ± 2 °C. **IR (cm⁻¹):** 3228, 1594, 1567, 1445,1308, 1223. **¹H NMR (400 MHz, MeOD):** δ 8.70 (d, *³J* = 4.7 Hz, 1H, H₁₄), 8.27 (d, *³J* = 7.9 Hz, 1H, H₁₁), 7.93 (td, *⁴J* = 1.4 Hz, *³J* = 7.9 Hz, 1H, H₁₂), 7.65 (m, 1H, H₄), 7.44 (d, *³J* = 8.3 Hz, 1H, H₇), 7.44 (d, *⁴J* = 1.4 Hz, *³J* = 7.9, 1H, H₁₃), 7.31 (d, *³J* = 8.3 Hz, 1H, H₆), 4.75 (s, 2H, CH₂). **¹³C NMR (101 MHz, Acetone-d6):** δ 151.3 (quat C, C₂), 149.4 (quat C, C₅), 149.4 (CH, C₁₄), 148.0 (quat C, C₁₀), 137.1 (CH, C₁₂), 137.1 (quat C, C₉), 136.5 (quat C, C₈), 124.5 (3x CH, C₄, C₇, C₁₃), 122.6 (CH, C₆), 121.1 (CH, C₁₁), 64.2 (CH₂). **LC-MS (ESI⁺)** *m*/*z* = 226.1 [M+H]⁺. The spectroscopic data are in accordance with those reported in literature (CAS # 308362-15-4).

### tert-Butyl 4-((4-(trifluoromethyl)phenyl)carbamoyl)piperazine-1-carboxylate (4a)

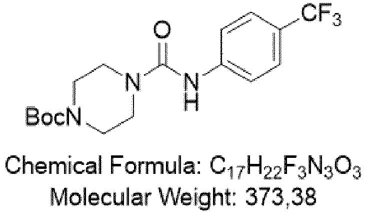

The title compound was prepared from Boc-piperazine (400 mg, 2.15 mmol, 1.0 eq.) and 4-(trifluoromethyl)phenyl isocyanate (0.34 mL, 2.36 mmol, 1.1 eq.) following the **general procedure (A).** Compound **4a** (780 mg, 2.09 mmol) was obtained as a white solid in 97% yield. **Rf=** 0.5 (CH₂Cl₂/MeOH, 96:4). **¹H NMR (250 MHz, DMSO-*d₆*):** δ 8.56 (s, 1H, NH), 7.54 (d, *³J* = 8.9 Hz, 2H, Hₐᵣ), 7.26 (d, *³J* = 8.9 Hz, 2H, Hₐᵣ), 3.60-3.44 (m, 8H, piperazine), 1.51 (s, 9H, -Boc). **LC-MS (ESI⁺)** *m*/*z* = 374.5 [M+H]⁺. The spectroscopic data are in accordance with those reported in literature (CAS # 651293-08-2).

### tert-Butyl 4-((3-(trifluoromethyl)phenyl)carbamoyl)piperazine-1-carboxylate (4b)

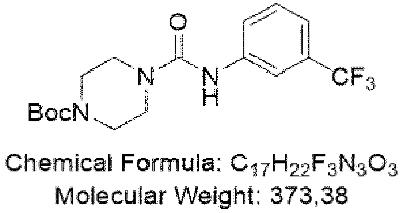

The title compound was prepared from Boc-piperazine (400 mg, 2.15 mmol, 1.0 eq.) and 3-(trifluoromethyl)phenyl isocyanate (0.34 mL, 2.36 mmol, 1.1 eq.) following the **general procedure (A).** Compound **4b** (763 mg, 2.04 mmol) was obtained as a white solid in 95% yield. **Rf=** 0.5 (CH₂Cl₂/MeOH, 96:4). **¹H NMR (250 MHz, DMSO-*d₆*):** δ 8.85 (s, 1H, NH), 7.86 (s, 1H, Hₐᵣ), 7.69 (d, *³J* = 7.7 Hz, 1H, Hₐᵣ), 7.42 (t, *³J* = 8.0 Hz, 1H, Hₐᵣ), 7.22 (dd, *⁴J* = 1.8 Hz, *³J* = 7.7 Hz, 1H, Hₐᵣ), 3.40 (m, 4H, piperazine), 3.28 (m, 4H, piperazine), 1.37 (s, 9H, -Boc). **LC-MS (ESI⁺)** *m*/*z* = 374.5 [M+H]⁺. The spectroscopic data are in accordance with those reported in literature (CAS # 1265591-99-8).

### tert-Butyl 4-((4-chlorophenyl)carbamoyl)piperazine-1-carboxylate (4c)

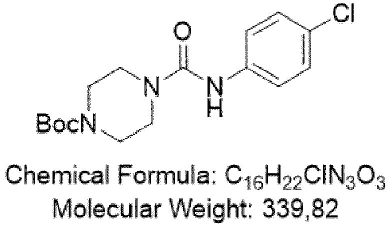

The title compound was prepared from Boc-piperazine (400 mg, 2.15 mmol, 1.0 eq.) and 4-chlorophenyl isocyanate (0.30 mL, 2.36 mmol, 1.1 eq.) following the **general procedure (A).** Compound **4c** (701 mg, 2.06 mmol) was obtained as a white solid in 96% yield. **Rf=** 0.43 (CH₂Cl₂/MeOH, 97:3). ¹H **NMR (250 MHz, DMSO-*d₆*):** δ 8.77 (s, 1H, NH), 7.77 (dd, *⁴J* = 1.5 Hz, *³J* = 8.1 Hz, 2H, Hₐᵣ), 7.42 (dd, *⁴J* = 1.5 Hz, *³J* = 8.1 Hz, 2H, Hₐᵣ), 3.60 (m, 4H, piperazine), 2.58 (m, 4H, piperazine), 1.40 (s, 9H, -Boc). **LC-MS (ESI⁺)** *m*/*z* = 340.9 [M+H]⁺. The spectroscopic data are in accordance with those reported in literature (CAS # 1315592-03-0).

### tert-butyl 4-((3-chlorophenyl)carbamoyl)piperazine-1-carboxylate (4d)

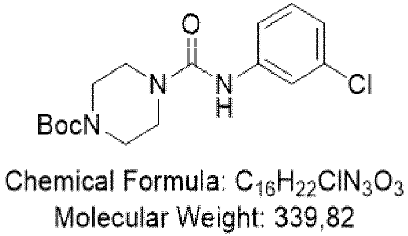

The title compound was prepared from Boc-piperazine (400 mg, 2.15 mmol, 1.0 eq.) and 3-chlorophenyl isocyanate (0.29 mL, 2.36 mmol, 1.1 eq.) following the **general procedure (A).** Compound **4d** (693 mg, 2.04 mmol) was obtained as a white solid in 95% yield. **Rf=** 0.43 (CH₂Cl₂/MeOH, 97:3). **¹H NMR (250 MHz, DMSO-*d₆*):** δ 8.83 (s, 1H, NH), 7.79 (d, *⁴J* = 1.7 Hz, 1H, Hₐᵣ), 7.60 (dt, *⁴J* = 1.7 Hz, *³J* = 8.2 Hz, 1H, Hₐᵣ), 7.35 (d, *³J* = 8.1 Hz, 1H, Hₐᵣ), 7.16 (dd, *⁴J* = 1.7 Hz, *³J* = 8.1 Hz, 1H, Hₐᵣ), 3.61 (m, 4H, piperazine), 3.58 (m, 4H, piperazine), 1.43 (s, 9H, -Boc). **LC-MS (ESI⁺)** *m*/*z* = 340.8 [M+H]⁺. The spectroscopic data are in accordance with those reported in literature (CAS # 1326562-27-9).

### tert-Butyl 4-((4-nitrophenyl)carbamoyl)piperazine-1-carboxylate (4e)

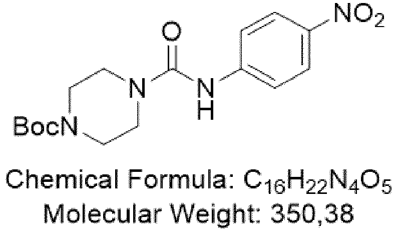

The title compound was prepared from Boc-piperazine (400 mg, 2.15 mmol, 1.0 eq.) and 4-nitrophenyl isocyanate (387 mg, 2.36 mmol, 1.1 eq.) following the **general procedure (A).** Compound **4e** (715 mg, 2.04 mmol) was obtained as a yellowish solid in 95% yield. **Rf=** 0.51 (CH₂Cl₂/MeOH, 95:5). **¹H NMR (250 MHz, DMSO-*d₆*):** δ 8.87 (s, 1H, NH), 8.13 (dd, *⁴J* = 1.8 Hz, *³J* = 8.7 Hz, 2H, Hₐᵣ), 7.36 (dd, *⁴J* = 2.3 Hz, *³J* = 8.7 Hz, 2H, Hₐᵣ), 3.60 (m, 4H, piperazine), 3.58 (m, 4H, piperazine), 1.39 (s, 9H, -Boc). **LC-MS (ESI⁺)** *m*/*z* = 351.4 [M+H]⁺. The spectroscopic data are in accordance with those reported in literature (CAS # 1315592-07-4).

### tert-butyl 4-((3-nitrophenyl)carbamoyl)piperazine-1-carboxylate (4f)

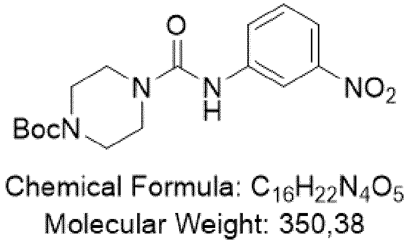

The title compound was prepared from Boc-piperazine (400 mg, 2.15 mmol, 1.0 eq.) and 3-chlorophenyl isocyanate (387 mg, 2.36 mmol, 1.1 eq.) following the **general procedure (A).** Compound **4f** (715 mg, 2.04 mmol) was obtained as a yellowish solid in 95% yield. **Rf=** 0.68 (CH₂Cl₂/MeOH, 9:1). **¹H NMR (250 MHz, DMSO-*d₆*):** δ 8.83 (s, 1H, NH), 7.89 (dd, *⁴J* = 1.7 Hz, *⁴J* = 1.5 Hz, 1H, Hₐᵣ), 7.49-7.41 (m, 3H, Hₐᵣ), 7.35 (d, *³J* = 8.1 Hz, 1H, Hₐᵣ), 7.16 (dd, *⁴J* = 1.7 Hz, *³J* = 8.1 Hz, 1H, Hₐᵣ), 3.60 (m, 4H, piperazine), 3.58 (m, 4H, piperazine), 1.41 (s, 9H, -Boc). **LC-MS (ESI⁺)** *m*/*z* = 351.5 [M+H]⁺. The spectroscopic data are in accordance with those reported in literature (CAS # 1324959-33-2).

### tert-butyl 4-(p-tolylcarbamoyl)piperazine-1-carboxylate (4g)

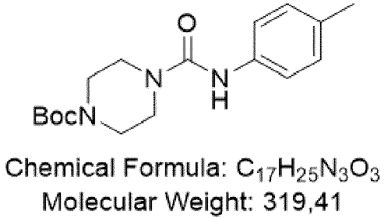

The title compound was prepared from Boc-piperazine (400 mg, 2.15 mmol, 1.0 eq.) and p-tolyl isocyanate (0.18 mL, 2.36 mmol, 1.1 eq.) following the **general procedure (A).** Compound **4g** (677 mg, 2.12 mmol) was obtained as a creamy solid in 98% yield. **Rf=** 0.43 (CH₂Cl₂/MeOH, 96:4). **¹H NMR (250 MHz, DMSO-*d₆*):** δ 8.53 (s, 1H, NH), 7.24 (d, *³J* = 8.2 Hz, 2H, Hₐᵣ), 7.10 (d, *³J* = 8.2 Hz, 2H, Hₐᵣ), 3.60 (m, 4H, piperazine), 3.58 (m, 4H, piperazine), 2.32 (s, 3H, CH₃), 1.42 (s, 9H, -Boc). **LC-MS (ESI⁺)** *m*/*z* = 320.4 [M+H]⁺. The spectroscopic data are in accordance with those reported in literature (CAS # 1325444-24-3).

### tert-butyl 4-((4-methoxyphenyl)carbamoyl)piperazine-1-carboxylate (4h)

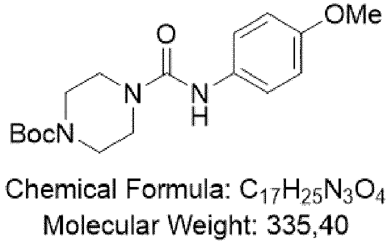

The title compound was prepared from Boc-piperazine (400 mg, 2.15 mmol, 1.0 eq.) and 4-methoxyphenyl isocyanate (0.31 mL, 2.36 mmol, 1.1 eq.) following the **general procedure (A).** Compound **4h** (711 mg, 2.12 mmol) was obtained as a white solid in 98% yield. **Rf=** 0.67 (CH₂Cl₂/MeOH, 9:1). **¹H NMR (250 MHz DMSO-*d₆*):** δ 7.23 (d, *³J* = 8.7 Hz, 2H, Hₐᵣ), 6.81 (d, *³J* = 8.7 Hz, 2H, Hₐᵣ), 6.72 (s, 1H, NH), 4.20-3.85 (m, 2H, piperazine), 3.77 (s, 3H, OCH3), 3.73-3.54 (m, 2H, piperazine), 3.53-3.18 (m, 4H, piperazine), 1.42 (s, 9H, -Boc). **LC-MS (ESI⁺)** *m*/*z* = 336.5 [M+H]⁺. The spectroscopic data are in accordance with those reported in literature (CAS # 1326394-37-9).

### tert-butyl 4-((4-(ethoxycarbonyl)phenyl)carbamoyl)piperazine-1-carboxylate (4i)

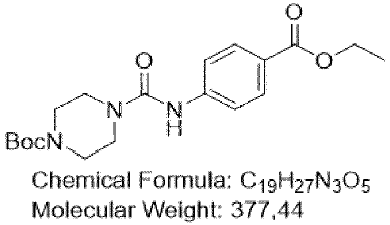

The title compound was prepared from Boc-piperazine (400 mg, 2.15 mmol, 1.0 eq.) and ethyl 4-isocyanatobenzoate (0.40 mL, 2.36 mmol, 1.1 eq.) following the **general procedure (A).** Compound **4i** (789 mg, 2.09 mmol) was obtained as a white solid in 97% yield. **Rf=** 0.67 (CH₂Cl₂/MeOH, 9:1). **¹H NMR (250 MHz, DMSO-*d₆*):** δ 8.05 (d, *³J* = 8.5 Hz, 2H, Hₐᵣ), 7.36 (d, *³J* = 8.5 Hz, 2H, Hₐᵣ), 6.80 (s, 1H, NH), 4.30 (m, 2H, CH₂), 4.20-3.85 (m, 2H, piperazine), 3.73-3.54 (m, 2H, piperazine), 3.53-3.18 (m, 4H, piperazine), 1.42 (s, 9H, -Boc), 1.29 (m, 3H, CH₃). **LC-MS (ESI⁺)** *m*/*z* = 378.5 [M+H]⁺. The spectroscopic data are in accordance with those reported in literature (CAS # 1459753-39-9).

### tert-butyl 4-(ethylcarbamoyl)piperazine-1-carboxylate (5a)

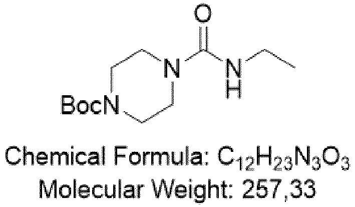

The title compound was prepared from Boc-piperazine (400 mg, 2.15 mmol, 1.0 eq.) and ethyl isocyanate (0.19 mL, 2.36 mmol, 1.1 eq.) following the **general procedure (A).** Compound **5** (530 mg, 2.06 mmol) was obtained as a white solid in 96% yield. **Rf=** 0.59 (CH₂Cl₂/MeOH, 9:1). **¹H NMR (250 MHz, DMSO-*d₆*):** δ 6.80 (s, 1H, NH), 4.30 (m, 2H, CH₂), 4.20-3.85 (m, 2H, piperazine), 3.73-3.54 (m, 2H, piperazine), 3.53-3.18 (m, 4H, piperazine), 3.09 (m, 2H, CH₂), 1.42 (s, 9H, -Boc), 1.23 (m, 3H, CH₃). **LC-MS (ESI⁺)** *m*/*z* = 258.4 [M+H]⁺. The spectroscopic data are in accordance with those reported in literature (CAS # 877165-63-4).

### tert-butyl 4-((2-ethoxy-2-oxoethyl)carbamoyl)piperazine-1-carboxylate (5b)

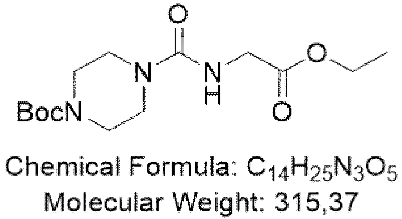

The title compound was prepared from Boc-piperazine (400 mg, 2.15 mmol, 1.0 eq.) and ethyl isocyanatoacetate (0.19 mL, 2.36 mmol, 1.1 eq.) following the **general procedure (A).** Compound **5b** (576 mg, 1.83 mmol) was obtained as a white solid in 85% yield. **Rf=** 0.76 (CH₂Cl₂/MeOH, 9:1). **¹H NMR (250 MHz, DMSO-*d₆*):** δ 6.80 (s, 1H, NH), 4.30 (m, 2H, CH₂), 4.20-3.85 (m, 4H, piperazine, O-CH₂), 3.72 (s, 2H, N-CH₂-CO), 3.70-3.54 (m, 2H, piperazine), 3.53-3.18 (m, 4H, piperazine), 1.42 (s, 9H, -Boc), 1.15 (m, 3H, CH₃). **LC-MS (ESI⁺)** *m*/*z* = 316.5 [M+H]⁺. The spectroscopic data are in accordance with those reported in literature (CAS # 470716-34-8).

### tert-butyl 4-((3-ethoxy-3-oxopropyl)carbamoyl)piperazine-1-carboxylate (5c)

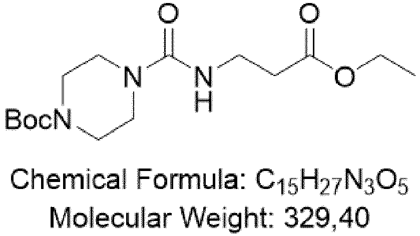

The title compound was prepared from Boc-piperazine (400 mg, 2.15 mmol, 1.0 eq.) and 3-isocyanato-propionic acid ethyl ester (0.31 mL, 2.36 mmol, 1.1 eq.) following the **general procedure (A).** Compound **5c** (687 mg, 2.09 mmol) was obtained as a white solid in 97% yield. **Rf=** 0.76 (CH₂Cl₂/MeOH, 9:1). **¹H NMR (250 MHz, DMSO-*d₆*):** δ 6.80 (s, 1H, NH), 4.30 (m, 2H, CH₂), 4.20-3.85 (m, 4H, piperazine, O-CH₂), 3.70-3.54 (m, 2H, piperazine), 3.53-3.18 (m, 8H, piperazine, N-CH₂, CH₂-CO), 1.43 (s, 9H, -Boc), 1.15 (m, 3H, CH₃). **LC-MS (ESI⁺)** *m*/*z* = 330.6 [M+H]⁺. The spectroscopic data are in accordance with those reported in literature (CAS # 2207360-04-9).

### N-(4-(trifluoromethyl)phenyl)piperazine-1-carboxamide (6a)

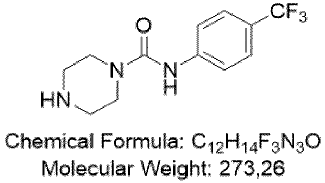

The title compound was prepared from **4a** (261 mg, 0.70 mmol, 1.0 eq.) and TFA (0.86 mL mL, 11.2 mmol, 16.0 eq.) following the **general procedure (B).** Compound **6a** (166 mg, 0.61 mmol) was obtained as a white solid in 87% yield. **Rf=** 0.14 (CH₂Cl₂/MeOH, 9:1). **¹H NMR (250 MHz, MeOD):** δ 7.54 (d, *³J* = 8.9 Hz, 2H, Hₐᵣ), 7.26 (d, *³J* = 8.9 Hz, 2H, Hₐᵣ), 3.60-3.44 (m, 8H, piperazine). **LC-MS (ESI⁺)** *m*/*z* = 274.4 [M+H]⁺. The spectroscopic data are in accordance with those reported in literature (CAS # 651293-08-2).

### N-(3-(trifluoromethyl)phenyl)piperazine-1-carboxamide (6b)

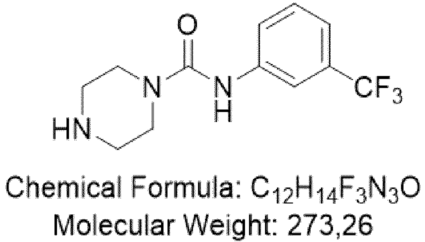

The title compound was prepared from **4b** (261 mg, 0.70 mmol, 1.0 eq.) and TFA (0.86 mL mL, 11.2 mmol, 16.0 eq.) following the **general procedure (B).** Compound **6b** (164 mg, 0.60 mmol) was obtained as a white solid in 85% yield. **Rf=** 0.14 (CH₂Cl₂/MeOH, 9:1). **¹H NMR (250 MHz, MeOD):** δ 7.86 (s, 1H, Hₐᵣ), 7.69 (d, *³J* = 7.7 Hz, 1H, Hₐᵣ), 7.42 (t, *³J* = 8.0 Hz, 1H, Hₐᵣ), 7.22 (dd, *⁴J* = 1.8 Hz, *³J* = 7.7 Hz, 1H, Hₐᵣ), 3.40 (m, 4H, piperazine), 3.28 (m, 4H, piperazine). **LC-MS (ESI⁺)** *m*/*z* = 274.4 [M+H]⁺. The spectroscopic data are in accordance with those reported in literature (CAS # 1225578-90-4).

### N-(4-chlorophenyl)piperazine-1-carboxamide (6c)

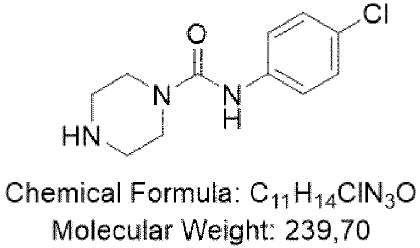

The title compound was prepared from **4c** (238 mg, 0.70 mmol, 1.0 eq.) and TFA (0.86 mL mL, 11.2 mmol, 16.0 eq.) following the **general procedure (B).** Compound **6c** (151 mg, 0.63 mmol) was obtained as a white solid in 90% yield. Rf= 0.14 (CH₂Cl₂/MeOH, 9:1). **Rf=** 0.34 (CH₂Cl₂/MeOH, 9:1). **¹H NMR (250 MHz, MeOD):** δ 7.77 (dd, *⁴J* = 1.5 Hz, *³J* = 8.1 Hz, 2H, Hₐᵣ), 7.42 (dd, *⁴J* = 1.5 Hz, *³J* = 8.1 Hz, 2H, Hₐᵣ), 3.60 (m, 4H, piperazine), 3.58 (m, 4H, piperazine). **LC-MS (ESI⁺)** *m*/*z* = 240.8 [M+H]⁺. The spectroscopic data are in accordance with those reported in literature (CAS # 923242-63-1).

### N-(3-chlorophenyl)piperazine-1-carboxamide (4d)

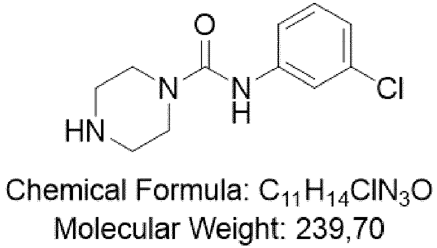

The title compound was prepared from **4d** (238 mg, 0.70 mmol, 1.0 eq.) and TFA (0.86 mL mL, 11.2 mmol, 16.0 eq.) following the **general procedure (B).** Compound **6d** (119 mg, 0.50 mmol) was obtained as a white solid in 71% yield. **Rf=** 0.23 (CH₂Cl₂/MeOH, 9:1). **¹H NMR (250 MHz, MeOD):** δ 7.79 (d, *⁴J* = 1.7 Hz, 1H, Hₐᵣ), 7.60 (dt, *⁴J* = 1.7 Hz, *³J* = 8.2 Hz, 1H, Hₐᵣ), 7.35 (d, *³J* = 8.1 Hz, 1H, Hₐᵣ), 7.16 (dd, *⁴J* = 1.7 Hz, *³J* = 8.1 Hz, 1H, Hₐᵣ), 3.61 (m, 4H, piperazine), 3.58 (m, 4H, piperazine. **LC-MS (ESI⁺)** *m*/*z* = 240.8 [M+H]⁺. The spectroscopic data are in accordance with those reported in literature (CAS # 923191-18-8).

### N-4-nitrohenlierazine-1-carboxamide (6e)

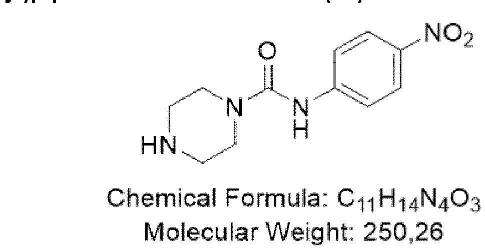

The title compound was prepared from **4e** (245 mg, 0.70 mmol, 1.0 eq.) and TFA (0.86 mL mL, 11.2 mmol, 16.0 eq.) following the **general procedure (B).** Compound **6e** (114 mg, 0.46 mmol) was obtained as a white solid in 65% yield. **Rf=** 0.11 (CH₂Cl₂/MeOH, 9:1). **¹H NMR(250 MHz, MeOD):** δ 8.13 (dd, *⁴J* = 1.8 Hz, *³J* = 8.7 Hz, 2H, Hₐᵣ), 7.36 (dd, *⁴J* = 2.3 Hz, *³J* = 8.7 Hz, 2H, Hₐᵣ), 3.60 (m, 4H, piperazine), 3.58 (m, 4H, piperazine). **LC-MS (ESI⁺)** *m*/*z* = 251.3 [M+H]⁺. The spectroscopic data are in accordance with those reported in literature (CAS # 1154396-59-4).

### N-(3-nitrophenyl)piperazine-1-carboxamide (6f)

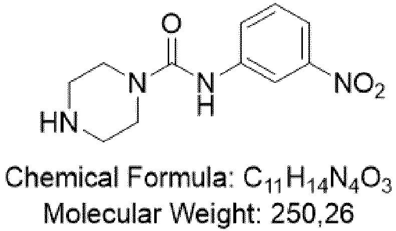

The title compound was prepared from **4f** (245 mg, 0.70 mmol, 1.0 eq.) and TFA (0.86 mL mL, 11.2 mmol, 16.0 eq.) following the **general procedure (B).** Compound **6f** (110 mg, 0.44 mmol) was obtained as a white solid in 65% yield. **Rf=** 0.11 (CH₂Cl₂/MeOH, 9:1). **¹H NMR (250 MHz, MeOD):** δ 7.89 (dd, *⁴J* = 1.7 Hz, *⁴J* = 1.5 Hz, 1H, Hₐᵣ), 7.49-7.41 (m, 3H, Hₐᵣ), 7.35 (d, *³J* = 8.1 Hz, 1H, Hₐᵣ), 7.16 (dd, *⁴J* = 1.7 Hz, *³J* = 8.1 Hz, 1H, Hₐᵣ), 3.60 (m, 4H, piperazine), 3.58 (m, 4H, piperazine). **LC-MS (ESI⁺)** *m*/*z* = 251.3 [M+H]⁺. The spectroscopic data are in accordance with those reported in literature (CAS # 1041516-73-7).

### N-(p-tolyl)piperazine-1-carboxamide (6g)

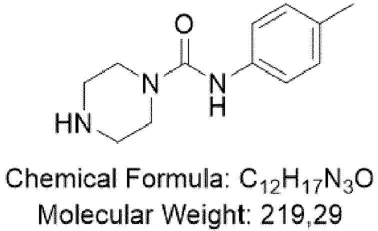

The title compound was prepared from **4g** (224 mg, 0.70 mmol, 1.0 eq.) and TFA (0.86 mL mL, 11.2 mmol, 16.0 eq.) following the **general procedure (B).** Compound **6g** (132 mg, 0.60 mmol) was obtained as a white solid in 86% yield. **Rf=** 0.22 (CH₂Cl₂/MeOH, 9:1). **¹H NMR (250 MHz, MeOD):** δ 7.24 (d, *³J* = 8.2 Hz, 2H, Hₐᵣ), 7.10 (d, *³J* = 8.2 Hz, 2H, Hₐᵣ), 3.60 (m, 4H, piperazine), 3.58 (m, 4H, piperazine), 2.32 (s, 3H, CH₃). **LC-MS (ESI⁺)** *m*/*z* = 220.4 [M+H]⁺. The spectroscopic data are in accordance with those reported in literature (CAS # 242814-90-0).

### N-(4-methoxyphenyl)piperazine-1-carboxamide (6h)

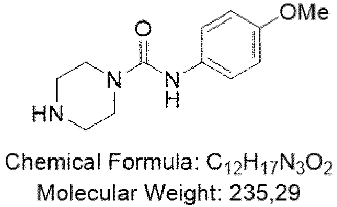

The title compound was prepared from **4h** (235 mg, 0.70 mmol, 1.0 eq.) and TFA (0.86 mL mL, 11.2 mmol, 16.0 eq.) following the **general procedure (B).** Compound **6h** (105 mg, 0.45 mmol) was obtained as a white solid in 64% yield. **Rf=** 0.23 (CH₂Cl₂/MeOH, 9:1). **¹H NMR (250 MHz, MeOD):** δ 7.23 (d, *³J* = 8.7 Hz, 2H, Hₐᵣ), 6.81 (d, *³J* = 8.7 Hz, 2H, Hₐᵣ), 4.20-3.85 (m, 2H, piperazine), 3.77 (s, 3H, OCH3), 3.73-3.54 (m, 2H, piperazine), 3.53-3.18 (m, 4H, piperazine). **LC-MS (ESI⁺)** *m*/*z* = 236.4 [M+H]⁺. The spectroscopic data are in accordance with those reported in literature (CAS # 79221-45-7).

### Ethyl-4-(piperazine-1-carboxamido)benzoate (6i)

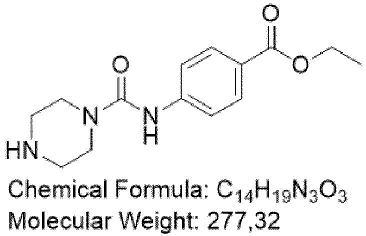

The title compound was prepared from **4i** (264 mg, 0.70 mmol, 1.0 eq.) and TFA (0.86 mL mL, 11.2 mmol, 16.0 eq.) following the **general procedure (B).** Compound **6i** (82 mg, 0.29 mmol) was obtained as a white solid in 42% yield. **Rf=** 0.08 (CH₂Cl₂/MeOH, 9:1). **¹H NMR (250 MHz, MeOD):** δ 8.05 (d, *³J* = 8.5 Hz, 2H, Hₐᵣ), 7.36 (d, *³J* = 8.5 Hz, 2H, Hₐᵣ), 4.30 (m, 2H, CH₂), 4.20-3.85 (m, 2H, piperazine), 3.73-3.54 (m, 2H, piperazine), 3.53-3.18 (m, 4H, piperazine), 1.29 (t, *³J* = 8.3 Hz, 3H, CH₃). **LC-MS (ESI⁺)** *m*/*z* = 378.5 [M+H]⁺. The spectroscopic data are in accordance with those reported in literature (CAS # 604768-15-2).

### N-ethylpiperazine-1-carboxamide (7a)

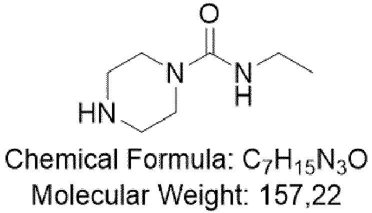

The title compound was prepared from **5a** (180 mg, 0.70 mmol, 1.0 eq.) and TFA (0.86 mL mL, 11.2 mmol, 16.0 eq.) following the **general procedure (B).** Compound **7a** (77 mg, 0.49 mmol) was obtained as a white solid in 70% yield. **Rf=** 0.11 (CH₂Cl₂/MeOH, 9:1). **¹H NMR (250 MHz, MeOD):** δ 4.30 (m, 2H, CH₂), 4.20-3.85 (m, 2H, piperazine), 3.73-3.54 (m, 2H, piperazine), 3.53-3.18 (m, 4H, piperazine), 3.09 (m, 2H, CH₂), 1.23 (m, 3H, CH₃). **LC-MS (ESI⁺)** *m*/*z* = 158.3 [M+H]⁺. The spectroscopic data are in accordance with those reported in literature (CAS # 75529-72-5).

### Ethyl-(piperazine-1-carbonyl)glycinate (7b)

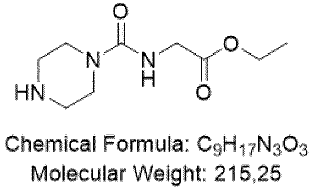

The title compound was prepared from **5b** (221 mg, 0.70 mmol, 1.0 eq.) and TFA (0.86 mL mL, 11.2 mmol, 16.0 eq.) following the **general procedure (B).** Compound **7b** (110 mg, 0.51 mmol) was obtained as a white solid in 73% yield. **Rf=** 0.11 (CH₂Cl₂/MeOH, 9:1). **¹H NMR (250 MHz, MeOD):** δ 4.30 (m, 2H, CH₂), 4.20-3.85 (m, 4H, piperazine, O-CH₂), 3.72 (s, 2H, N-CH₂-CO), 3.70-3.54 (m, 2H, piperazine), 3.53-3.18 (m, 4H, piperazine), 1.15 (t, *³J* = 8.1 Hz, 3H, CH₃). **LC-MS (ESI⁺)** *m*/*z* = 216.3 [M+H]⁺. The spectroscopic data are in accordance with those reported in literature (CAS # 1247702-56-2).

### Ethyl-3-(piperazine-1-carboxamido)propanoate (7c)

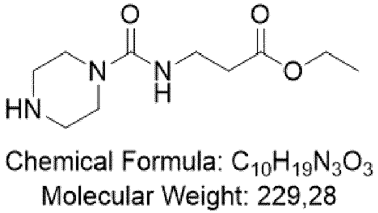

The title compound was prepared from **5c** (231 mg, 0.70 mmol, 1.0 eq.) and TFA (0.86 mL, 11.2 mmol, 16.0 eq.) following the **general procedure (B).** Compound **7c** (142 mg, 0.62 mmol) was obtained as a white solid in 89% yield. **Rf=** 0.11 (CH₂Cl₂/MeOH, 9:1). **¹H NMR (250 MHz, MeOD):** δ 4.30 (m, 2H, CH₂), 4.20-3.85 (m, 4H, piperazine, O-CH₂), 3.70-3.54 (m, 2H, piperazine), 3.53-3.18 (m, 8H, piperazine, N-CH₂, CH₂-CO), 1.15 (t, *³J* = 8.5 Hz, 3H, CH₃). **LC-MS (ESI⁺)** *m*/*z* = 230.3 [M+H]⁺. The spectroscopic data are in accordance with those reported in literature (CAS # 2166682-33-1).

### 4-((2-(pyridin-2-yl)-1H-benzo[d]imidazol-5-yl)methyl)-N-(4-(trifluoromethyl)phenyl)piperazine-1-carboxamide (I-52)

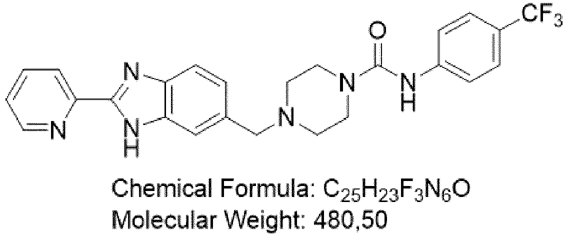

The title compound was prepared from **3** (200 mg, 0.89 mmol, 1.0 eq.) and **6a** (243 mg, 0.89 mmol, 1.0 eq.) following the **general procedure (C).** Compound **I-52** (248 mg, 0.51 mmol) was obtained as an orange solid in 58% yield. **Rf=** 0.39 (CH₂Cl₂/MeOH, 9:1). **Mp=** 136 ± 2 °C. **IR (cm⁻¹):** 1647, 1598, 1526, 1446, 1418, 1320, 1244, 1183, 1160, 11100, 1064. **¹H NMR (400 MHz, Acetone-*d₆*):** δ 12.04 (br s, 1H, -NHₐᵣ), 8.62 (d, *³J* = 4,6 Hz, 1H, Hₐᵣ), 8.37 (d, *³J* = 7.8 Hz, 1H, Hₐᵣ), 8.32 (s, 1H, -CONH), 7.93 (td, *⁴J* = 1.8 Hz, *³J* = 7.8 Hz, 1H, Hₐᵣ), 7.71 (d, *³J* = 8.6 Hz, 2H, Hₐᵣ), 7.61 (m, 2H, Hₐᵣ), 7.51 (d, *³J* = 8.6 Hz, 2H, Hₐᵣ), 7.42 (ddd, *⁴J* = 1.1 Hz, *⁴J* = 4.8 Hz , *³J* = 7.8 Hz, 1H, Hₐᵣ), 7.25 (d, *³J* = 8.2 Hz, 1H, Hₐᵣ), 3.66 (s, 2H, CH₂), 3.55 (m, 4H, CH₂-piperazine), 2.49 (m, 4H, CH₂-piperazine). ¹³C **NMR (101 MHz, Acetone-*d₆*):** δ 154.9 (quat C), 154.4 (quat C), 151.2 (quat C), 149.8 (quat C, CH), 149.0 (quat C), 144.5 (quat C), 144.4 (quat C), 137.1 (CH, quat C), 125.6 (2x CH), 125.5 (2x CH), 124.4 (CH), 121.3 (CH), 118.8 (2x CH), 118.7 (CH), 62.8 (CH₂), 52.7 (2x CH₂, Cₚᵢₚₑᵣ.), 44.0 (2x CH₂, Cₚᵢₚₑᵣ.). **HRMS (ESI⁺)** calcd. for C₂₅H₂₄F₃N₆O, 481,1958 [M+H]⁺; found, 481.1954.

### 4-((2-(pyridin-2-yl)-1H-benzo[d]imidazol-5-yl)methyl)-N-(3-(trifluoromethyl)phenyl)piperazine-1-carboxamide (II-1)

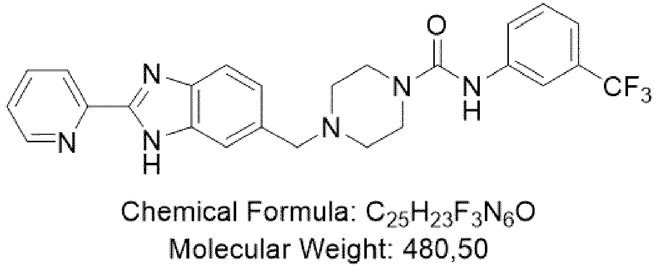

The title compound was prepared from **3** (200 mg, 0.89 mmol, 1.0 eq.) and **6b** (243 mg, 0.89 mmol, 1.0 eq.) following the **general procedure (C).** Compound **11-1** (218 mg, 0.45 mmol) was obtained as an orange solid in 51% yield. **Rf=** 0.40 (CH₂Cl₂/MeOH, 9:1). **Mp=** 139 ± 2 °C. **IR (cm⁻¹):** 1647, 1598, 1526, 1446, 1418, 1320, 1244, 1183, 1160, 11100, 1064. **¹H NMR (400 MHz, MeOD):** δ 8.69 (d, *³J* = 4,9 Hz, 1H, Hₐᵣ), 8.31 - 8.19 (m, 1H, Hₐᵣ), 7.93 (t, *³J* = 7.8 Hz, 1H, Hₐᵣ), 7.74 (m, 1H, Hₐᵣ), 7.57 (m, 3H, Hₐᵣ), 7.50 - 7.22 (m, 4H, Hₐᵣ), 3.68 (s, 2H, CH₂), 3.55 (m, 4H, CH₂-piperazine), 2.53 (m, 4H, CH₂-piperazine). ¹³C **NMR (101 MHz, MeOD):** δ 156.0 (2x quat C), 151.5 (quat C), 149.5 (CH), 148.0 (quat C), 140.6 (quat C), 137.1 (CH, quat C), 129.0 (2x CH), 124.5 (CH), 123.3 (CH), 121.1 (CH), 118.6 (2x CH), 116.6 (2x CH), 116.5 (2x CH), 62.7 (CH₂), 52.5 (2x CH₂, Cₚᵢₚₑᵣ.) , 43.6 (2x CH₂, Cₚᵢₚₑᵣ.). **HRMS (ESI⁺)** calcd. for C₂₅H₂₄F₃N₆O, 481,1958 [M+H]⁺; found, 481.1952.

### N-(4-chlorophenyl)-4-((2-(pyridin-2-yl)-1H-benzo[d]imidazol-6-yl)methyl)piperazine-1-carboxamide (II-2)

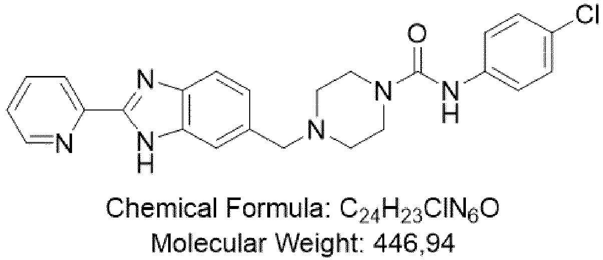

The title compound was prepared from **3** (200 mg, 0.89 mmol, 1.0 eq.) and **6c** (213 mg, 0.89 mmol, 1.0 eq.) following the **general procedure (C).** Compound **II-2** (219 mg, 0.49 mmol) was obtained as an orange solid in 55% yield. **Rf=** 0.45 (CH₂Cl₂/MeOH, 9:1). **Mp=** 210 ± 2 °C. **IR (cm⁻¹):** 1636, 1592, 1530, 1496, 1445, 1424, 1392, 1285, 1247, 1151, 1087. **¹H NMR (250 MHz, DMSO-*d₆*):** δ 13.05 (s, 1H, NHₐᵣ), 8.73 (d, *³J* = 4.4 Hz, 1H, Hₐᵣ), 8.62 (s, 1H, CO-NH), 8.32 (d, *³J* = 7.9 Hz, 1H, Hₐᵣ), 8.00 (td, *⁴J* = 1.4 Hz, *³J* = 7.7 Hz, 1H, Hₐᵣ), 7.64 (m, 1H, Hₐᵣ), 7.49 (m, 4H, Hₐᵣ), 7.23 (m, 3H, Hₐᵣ), 3.61 (s, 2H, CH₂), 3.46 (m, 4H, CH₂-piperazine), 2.42 (m, 4H, CH₂-piperazine). **¹³C NMR (101 MHz, DMSO-*d₆*):** δ 155.2 (quat C), 151.3 (quat C), 149.8 (quat C, CH), 149.0 (quat C), 143.7 (quat C), 140.7 (quat C), 138.0 (CH), 135.5 (quat C), 128.6 (2x CH), 125.7 (quat C), 125.1 (CH), 123.8 (CH), 121.8 (CH), 121.4 (2x CH), 119.3 (CH), 112.1 (CH), 62.9 (CH₂), 53.0 (2x CH₂, Cₚᵢₚₑᵣ.), 44.2 (2x CH₂, Cₚᵢₚₑᵣ.). **HRMS (ESI⁺)** calcd. for C₂₄H₂₄ClN₆O, 447.1695 [M+H]⁺; found, 447.1696.

### N-(3-chlorophenyl)-4-((2-(pyridin-2-yl)-1H-benzo[d]imidazol-5-yl)methyl)piperazine-1-carboxamide (II-3)

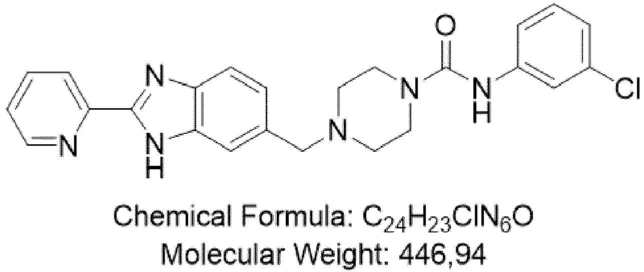

The title compound was prepared from **3** (200 mg, 0.89 mmol, 1.0 eq.) and **6d** (213 mg, 0.89 mmol, 1.0 eq.) following the **general procedure (C).** Compound **II-3** (278 mg, 0.62 mmol) was obtained as an orange solid in 70% yield. **Rf=** 0.24 (CH₂Cl₂/MeOH, 9:1). **Mp=** 118 ± 2 °C. **IR (cm⁻¹):** 1640, 1559, 1527, 1482, 1445, 1425, 1404, 1334, 1302, 1270, 1231, 1146, 1114. **¹H NMR (400 MHz, Acetone-*d₆*):** δ 12.08 (br s, 1H, NHₐᵣ), 8.62 (d, *³J =* 4.2 Hz, 1H, Hₐᵣ), 8.37 (d, *³J* = 7.9 Hz, 1H, Hₐᵣ), 8.16 (s, 1H, NH), 7.92 (td, *⁴J* = 1.7 Hz, *³J =* 7.7 Hz, 1H, Hₐᵣ), 7.73 (s, 1H, Hₐᵣ), 7.62 (m, 2H, Hₐᵣ), 7.40 (m, 2H, Hₐᵣ), 7.25 (m, 1H, Hₐᵣ), 7.16 (t, *³J* = 8.1 Hz, 1H, Hₐᵣ), 6.90 (dd, *⁴J* = 1.2 Hz, *³J* = 7.9 Hz, 1H, Hₐᵣ), 3.64 (s, 2H, CH₂), 3.54 (m, 4H, CH₂-piperazine), 2.57 (m, 4H, CH₂-piperazine). **¹³C NMR (100 MHz, Acetone-*d₆*):** δ 154.6 (quat C), 154.6 (quat C), 151.2 (quat C), 149.4 (CH, quat C), 149.0 (quat C), 142.4 (quat C), 142.3 (quat C), 137.1 (CH), 133.5 (quat C), 129.7 (CH), 124.4 (CH), 121.4 (2x CH), 121.3 (CH), 119.0 (CH), 118.9 (CH), 117.4 (CH), 117.3 (CH), 62.8 (CH₂), 52.7 (2x CH₂, Cₚᵢₚₑᵣ.), 44.0 (2x CH₂, Cₚᵢₚₑᵣ.). **HRMS (ESI⁺)** calcd. for C₂₄H₂₄ClN₆O, 447.1695 [M+H]⁺; found, 447.1695.

### N-(4-nitrophenyl)-4-((2-(pyridin-2-yl)-1H-benzo[d]imidazol-5-yl)methyl)piperazine-1-carboxamide (II-4)

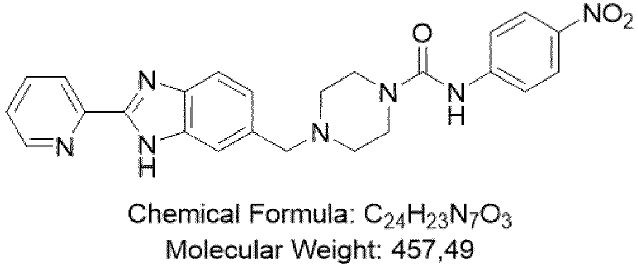

The title compound was prepared from **3** (200 mg, 0.89 mmol, 1.0 eq.) and **6e** (312 mg, 0.89 mmol, 1.0 eq.) following the **general procedure (C).** Compound **II-4** (264 mg, 0.58 mmol) was obtained as an orange solid in 65% yield. **Rf=** 0.41 (CH₂Cl₂/MeOH, 9:1). **Mp=** 142 ± 2 °C. **IR (cm⁻¹):** 1651, 1595, 1541, 1499, 1418, 1326, 1300, 1231, 1178, 1146, 1100. **¹H NMR (400 MHz, Acetone-*d₆*):** δ 12.06 (br s, 1H, NH), 8.68 (d, *³J* = 4.7 Hz, 1H, Hₐᵣ), 8.62 (s, 1H, CO-NH), 8.42 (d, *³J* = 7.9 Hz, 1H, Hₐᵣ), 8.15 (d, *³J* = 8.2 Hz, 2H, Hₐᵣ), 7.98 (td, *⁴J* = 1.4 Hz, *³J* = 7.7 Hz, 1H, Hₐᵣ), 7.77 (d, *³J* = 8.2 Hz, 2H, Hₐᵣ), 7.64 (m, 2H, Hₐᵣ), 7.46 (m, 1H, Hₐᵣ), 7.29 (m, 1H), 3.66 (s, 2H, CH₂), 3.61 (m, 4H, CH₂-piperazine), 2.52 (m, 4H, CH₂-piperazine). **¹³C NMR (101 MHz, Acetone-*d₆*):** δ 154.0 (quat C), 153.9 (quat C), 151.1 (quat C), 149.4 (CH), 149.1 (quat C), 147.4 (quat C), 142.0 (quat C), 144.1 (quat C), 137.1 (CH), 133.7 (quat C), 124.7 (CH), 124.5 (2x CH), 123.4 (CH), 121.2 (CH), 120.0 (CH), 119.27 (CH), 118.2 (2x CH), 62.9 (CH₂), 52.7 (2x CH₂, Cₚᵢₚₑᵣ.), 44.2 (2x CH₂, Cₚᵢₚₑᵣ.). **HRMS (ESI⁺)** calcd. for C₂₄H₂₄N₇O₃, 458.1935 [M+H]⁺; found, 458.1933.

### N-(3-nitrophenyl)-4-((2-(pyridin-2-yl)-1H-benzo[d]imidazol-5-yl)methyl)piperazine-1-carboxamide (II-5)

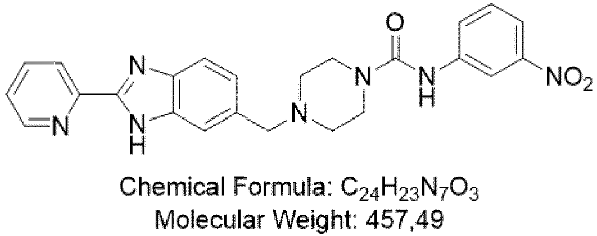

The title compound was prepared from **3** (200 mg, 0.89 mmol, 1.0 eq.) and **6f** (312 mg, 0.89 mmol, 1.0 eq.) following the **general procedure (C).** Compound **II-5** (256 mg, 0.56 mmol) was obtained as an orange solid in 63% yield. **Rf=** 0.41 (CH₂Cl₂/MeOH, 9:1). **Mp=** 152 ± 2 °C. **IR (cm⁻¹):** 1668, 1596, 1526, 1433, 1348, 1300, 1244, 1200, 1178, 1127. **¹H NMR (400 MHz, Acetone-*d₆*):** δ 12.31 (br s, 1H, NHₐᵣ), 8.69 (d, *³J* = 7.5 Hz, 1H, Hₐᵣ), 8.53 (m, 2H, CO-NH, Hₐᵣ), 8.37 (d, *³J* = 7.9 Hz, 1H, Hₐᵣ), 7.98-7.87 (m, 2H, Hₐᵣ), 7.75 (m, 1H, Hₐᵣ), 7.62 (m, 2H, Hₐᵣ), 7.49-7.38 (m, 2H, Hₐᵣ), 7.24 (d, *³J* = 7.7 Hz, 1H, Hₐᵣ), 3.64 (s, 2H, CH₂), 3.57 (m, 4H, CH₂-piperazine), 2.49 (m, 4H, CH₂-piperazine). **¹³C NMR (101 MHz, Acetone-*d₆*):** δ 154.2 (quat C), 153.7 (quat C), 150.9 (quat C), 149.2 (CH), 149.1 (quat C), 147.4 (quat C), 142.0 (quat C), 144.1 (quat C), 137.1 (CH), 133.7 (quat C), 124.7 (CH), 124.4 (2x CH), 123.4 (CH), 121.2 (CH), 120.0 (CH), 119.27 (CH), 118.2 (2x CH), 62.9 (CH₂), 52.6 (2x CH₂, Cₚᵢₚₑᵣ.), 44.1 (2x CH₂, C_{piper.}). HRMS (ESI⁺) calcd. for C₂₄H₂₄N₇O₃, 458.1935 [M+H]⁺; found, 458.1935.

### 4-((2-(pyridin-2-yl)-1H-benzo[d]imidazol-5-yl)methyl)-N-(p-tolyl)piperazine-1-carboxamide (I-48)

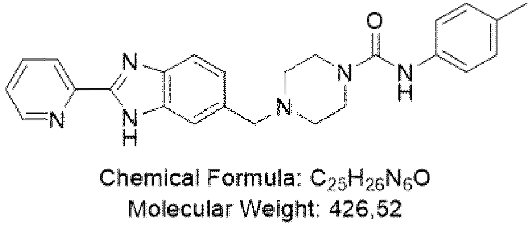

The title compound was prepared from **3** (200 mg, 0.89 mmol, 1.0 eq.) and **6g** (312 mg, 0.89 mmol, 1.0 eq.) following the **general procedure (C).** Compound **I-48** (256 mg, 0.56 mmol) was obtained as an orange solid in 86% yield. **Rf=** 0.40 (CH₂Cl₂/MeOH, 9:1). **Mp=** 170 ± 2 °C. **IR (cm⁻¹):** 1640, 1513, 1445, 1408, 1310, 1299, 1146, 1111, 1001. **¹H NMR (400 MHz, Acetone-*d₆*):** δ 12.35 (br s, 1H, NHₐᵣ), 8.68 (d, *³J* = 4.7 Hz, 1H, Hₐᵣ), 8.42 (d, *³J* = 7.9 Hz, 1H, Hₐᵣ), 7.98 (t, *³J* = 7.6 Hz, 1H, Hₐᵣ), 7.77-7.65 (m, 2H, Hₐᵣ), 7.47 (m,, 1H, Hₐᵣ), 7.41 (m, 1H, Hₐᵣ), 7.30 (d, *³J* = 8.0 Hz, Hₐᵣ), 7.03 (d, *³J* = 8.3 Hz, Hₐᵣ), 3.68 (s, 2H, CH₂), 3.55 (m, 4H, CH₂-piperazine), 2.50 (m, 4H, CH₂-piperazine), 2.28 (s, 3H, CH₃). ¹³C **NMR (100 MHz, Acetone-*d₆*):** δ 155.1 (quat C), 155.0 (quat C), 149.4 (CH), 149.1 (quat C), 138.2 (quat C), 138.1 (quat C), 137.1 (quat C), 130.9 (quat C), 130.9 (quat C), 128.7 (4x CH), 124.4 (CH), 121.3 (CH), 119.6 (2x CH), 119.5 (2x CH), 62.9 (CH₂), 52.9 (2x CH₂, Cₚᵢₚₑᵣ.), 44.0 (2x CH₂, Cₚᵢₚₑᵣ.), 19.8 (CH₃). **HRMS (ESI⁺)** calcd. for C₂₅H₂₇N₇O, 427.2241 [M+H]⁺; found, 427.2242.

### N-(4-methoxyphenyl)-4-((2-(pyridin-2-yl)-1H-benzo[d]imidazol-5-yl)methyl)piperazine-1-carboxamide (II-6)

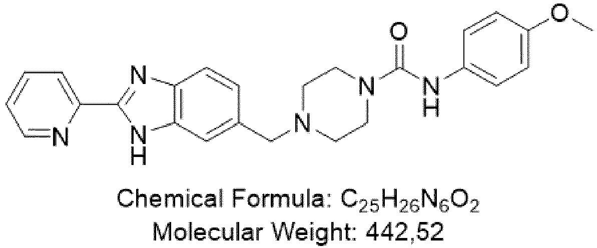

The title compound was prepared from **3** (200 mg, 0.89 mmol, 1.0 eq.) and **6h** (298 mg, 0.89 mmol, 1.0 eq.) following the **general procedure (C).** Compound **II-6** (256 mg, 0.71 mmol) was obtained as an orange solid in 80% yield. **Rf=** 0.43 (CH₂Cl₂/MeOH, 9:1). **Mp=** 124 ± 2 °C. **IR (cm⁻¹):** 1632, 1509, 1444, 1417, 1295, 1228, 1178, 1146, 1109, 1033. **¹H NMR (250 MHz, DMSO-*d₆*):** δ 13.06 (s, 1H, NH), 8.73 (d, *³J* = 4.5 Hz, 1H, Hₐᵣ), 8.32 (m, 2H, CO-NH, Hₐᵣ), 8.00 (td, *⁴J* = 1.4 Hz, *³J* = 7.7 Hz, 1H, Hₐᵣ), 7.64 (m, 1H, Hₐᵣ), 7.52 (m, 2H, Hₐᵣ), 7.34 (d, *³J* = 9.0 Hz, 2H, Hₐᵣ), 7.22 (m, 1H, Hₐᵣ), 6.81 (d, *³J* = 9.0 Hz, 2H, Hₐᵣ), 3.70 (s, 3H, OCH₃), 3.63 (s, 2H, CH₂), 3.45 (m, 4H, CH₂-piperazine), 2.43 (m, 4H, CH₂-piperazine). **¹³C NMR (101 MHz, DMSO-*d₆*):** δ 155.7 (quat C), 154.9 (quat C), 151.4 (quat C), 149.8 (CH), 149.0 (quat C), 144.4 (quat C), 138.0 (quat C), 135.5 (quat C), 133.9 (quat C), 125.1 (CH), 123.9 (CH), 122.0 (CH), 121.8 (CH), 119.3 (CH), 114.0 (CH), 112.1 (CH), 62.9 (CH₂), 55.6 (CH₃), 53.0 (2x CH₂, Cₚᵢₚₑᵣ.), 44.2 (2x CH₂, Cₚᵢₚₑᵣ.). **HRMS** (ESI⁺) calcd. for C₂₅H₂₇N₆O₂, 443.2190 [M+H]⁺; found, 443.2193.

### N-(4-aminophenyl)-4-((2-(pyridin-2-yl)-1H-benzo[d]imidazol-5-yl)methyl)piperazine-1-carboxamide (II-7)

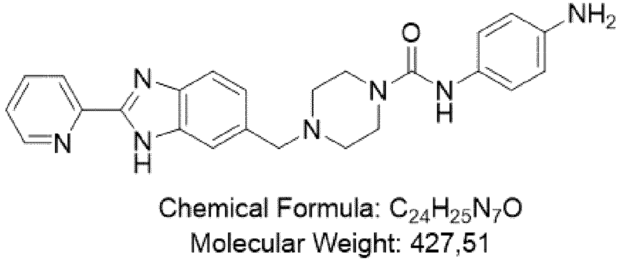

To a solution of compound **8e** (70 mg, 0.15 mmol, 1.0 eq.) in MeOH/ CH₂Cl₂ (5:1) was added Pd/C 10% (32 mg, 0.03 mmol, 20 mol%). The reaction medium was purged with hydrogen gas and stirred for 3 hours at room temperature under atmospheric pressure. The reaction mixture was filtered over Celite and concentrated *in vacuo.* The crude residue was purified by flash-column chromatography (silica gel, CH₂Cl₂/MeOH, 95:5) to give the compound **II-7** (43 mg, 0.10 mmol) as a brown solid in 70% yield. **Rf=** 0.12 (CH₂Cl₂/MeOH, 9:1). **Mp=** 150 ± 2 °C. **IR (cm⁻¹):** 3227, 2918, 1704, 1628, 1594, 1514, 1431, 1403, 1363, 1304, 1242, 1145. **¹H NMR (400 MHz, MeOD):** δ 8.74 (d, ³*J*=4.5 Hz, 1H, Hₐᵣ), 8.30 (d, ³*J*=7.9 Hz, 1H, Hₐᵣ), 7.98 (td, *⁴J* = 1.7 Hz, *³J* =7.7 Hz, 1H, Hₐᵣ), 7.66 (m, 2H, Hₐᵣ), 7.49 (m, 1H, Hₐᵣ), 7.34 (d, *³J* = 8.2 Hz, 1H, Hₐᵣ), 7.07 (d, *³J* = 8.8 Hz, 2H, Hₐᵣ), 6.70 (d, *³J* = 8.8 Hz, 2H, Hₐᵣ) 3.72 (s, 2H, CH₂), 3.55 (m, 4H, CH₂-piperazine), 2.55 (m, 4H, CH₂-piperazine). **¹³C NMR (101 MHz, MeOD):** δ 157.3 (2x quat C), 151.5 (quat C), 149.5 (quat C, CH), 148.0 (quat C), 143.6 (2x quat C), 137.1 (CH), 130.1 (quat C), 124.6 (CH), 123.6 (4x CH), 121.1 (CH), 115.5 (4x CH), 62.7 (CH₂), 52.5 (2x CH₂, Cₚᵢₚₑᵣ.), 43.5 (2x CH₂, Cₚᵢₚₑᵣ.). **HRMS (ESI⁺)** calcd. for C₂₄H₂₆N₇O, 428.2193 [M+H]⁺; found, 428.2193.

### Ethyl-4-(4-((2-(pyridin-2-yl)-1H-benzo[d]imidazol-5-yl)methyl)piperazine-1-carboxamido)benzoate (II-8)

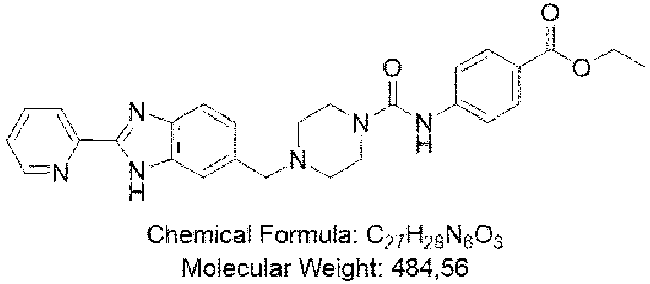

The title compound was prepared from **3** (200 mg, 0.89 mmol, 1.0 eq.) and **7a** (245 mg, 0.89 mmol, 1.0 eq.) following the **general procedure (C).** Compound II-8 (131 mg, 0.27 mmol) was obtained as an orange solid in 30% yield. **Rf=** 0.43 (CH₂Cl₂/MeOH, 9:1). Mp: 158 ± 2 °C. **IR (cm⁻¹):** 1705, 1645, 1593, 1516, 1446, 1416, 1366, 1308, 1276, 1240, 1173, 1147, 1103. **¹H NMR (250 MHz, DMSO-*d₆*):** δ 13.05 (s, 1H, NHₐᵣ), 8.89 (s, 1H, CO-NH), 8.73 (d, *³J* = 4.6 Hz, 1H, Hₐᵣ), 8.32 (d, *³J* = 7.9 Hz, 1H, Hₐᵣ), 8.00 (td, *³J* = 1.2 Hz, *³J* = 7.7 Hz, 1H, Hₐᵣ), 7.83 (d, *³J* = 8.7 Hz, 2H, Hₐᵣ), 7.63 (m, 3H, Hₐᵣ), 7.51 (m, 2H, Hₐᵣ), 7.21 (m, 1H, Hₐᵣ), 4.27 (q, *³J* = 7.0 Hz, 2H, CH₂), 3.63 (s, 2H, CH₂), 3.49 (m, 4H, CH₂-piperazine), 2.44 (m, 4H, CH₂-piperazine), 1.30 (t, *³J* = 7.0 Hz, 3H, CH₃). **¹³C NMR (101 MHz, DMSO-*d₆*):** δ 166.0 (quat C), 154.8 (quat C), 151.3 (2x quat C), 149.8 (CH), 149.0 (quat C), 145.8 (quat C), 144.4 (quat C), 143.7 (quat C), 138.0 (CH), 135.5 (quat C), 130.3 (2x CH), 125.1 (CH), 122.9 (CH), 121.8 (CH), 118.8 (2x CH), 112.7 (CH), 112.1 (CH), 62.9 (CH₂), 60.7 (CH2), 53.0 (2x CH₂, Cₚᵢₚₑᵣ.), 44.3 (2x CH₂, Cₚᵢₚₑᵣ.), 14.7 (CH₃). **HRMS (ESI⁺)** calcd. for C₂₇H₂₉N₆O₃, 485.2296 [M+H]⁺; found, 485.2296.

### Ethyl-3-(4-((2-(pyridin-2-yl)-1H-benzo[d]imidazol-6-yl)methyl)piperazine-1-carboxamido)propanoate (II-9)

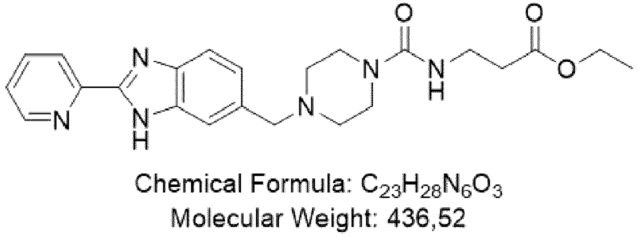

The title compound was prepared from **3** (200 mg, 0.89 mmol, 1.0 eq.) and **7b** (204 mg, 0.89 mmol, 1.0 eq.) following the **general procedure (C).** Compound **II-9** (282 mg, 0.67 mmol) was obtained as an orange solid in 75% yield. **Rf=** 0.43 (CH₂Cl₂/MeOH, 9:1). **Mp=** 104 ± 2 °C. **IR (cm⁻¹):** 1728, 1621, 1540, 1445, 1398, 1372, 1314, 1261, 1182, 1149, 1027. **¹H NMR (400 MHz, Acetone-*d₆*):** δ 12.10 (s, 1H, NH), 8.66 (d, *³J* = 4.6 Hz, 1H, Hₐᵣ), 8.40 (d, *³J* = 7.9 Hz, 1H, Hₐᵣ), 7.96 (t, *³J* = 7.7, 1H, Hₐᵣ), 7.63 (m, 2H, Hₐᵣ), 7.45 (m, 1H, Hₐᵣ), 7.26 (m, 1H, Hₐᵣ), 6.01 (s, 1H, NH), 4.07 (q, *³J* = 7.1 Hz, 2H, CH₂), 3.62 (s, 2H, CH₂), 3.39 (m, 6H, CH₂, CH₂-piperazine), 2.50 (t, *³J* = 6.8 Hz, 2H, CH₂), 2.41 (m, 4H, CH₂-piperazine), 1.20 (t, *³J* = 7.1 Hz, 3H, CH₃). **¹³C NMR (101 MHz, Acetone-*d₆*):** δ 171.7 (quat C), 157.5 (quat C), 151.1 (quat C), 149.4 (CH), 149.1 (quat C), 144.1 (quat C), 137.1 (CH), 135.0 (quat C), 133.9 (quat C), 124.3 (CH), 123.4 (CH), 121.3 (CH), 119.2 (CH), 112.0 (CH), 63.0 (CH₂), 59.7 (CH₂), 52.8 (2x CH₂, Cₚᵢₚₑᵣ.), 43.8 (2x CH₂, Cₚᵢₚₑᵣ.), 36.6 (CH₂), 34.8 (CH₂), 13.7 (CH₃). **HRMS (ESI⁺)** calcd. for C₂₃H₂₉N₆O₃, 437.2296 [M+H]⁺; found, 437.2296.

### tert-Butyl 4-((4-(trifluoromethyl)benzyl)carbamoyl)piperazine-1-carboxylate (9)

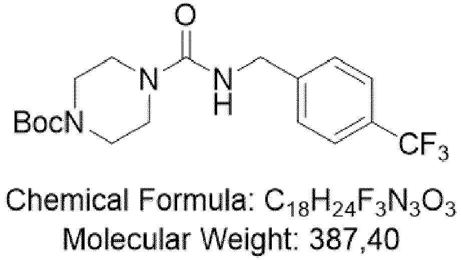

The title compound was prepared from Boc-piperazine (400 mg, 2.15 mmol, 1.0 eq.) and 4-(trifluoromethyl)benzyl isocyanate (0.39 mL, 2.36 mmol, 1.1 eq.) following the **general procedure (A).** Compound **9** (816 mg, 2.11 mmol) was obtained as a white solid in 98% yield. **Rf=** 0.55 (CH₂Cl₂/MeOH, 9:1). **¹H NMR (250 MHz, DMSO-*d₆*):** δ 7.64 (dd, *⁴J* = 1.9 Hz, *³J* = 8.5 Hz, 2H, Hₐᵣ), 7.54 (dd, *⁴J* = 1.4 Hz, *³J* = 8.5 Hz, 2H, Hₐᵣ), 6.94 (s, 1H, NH), 4.48 (s, 2H, CH₂), 3.59 (m, 4H, piperazine), 3.37 (m, 4H, piperazine), 1.43 (s, 9H, -Boc). **LC-MS (ESI⁺)** *m*/*z* = 388.4 [M+H]⁺.

### N-(4-(trifluoromethyl)benzyl)piperazine-1-carboxamide (10)

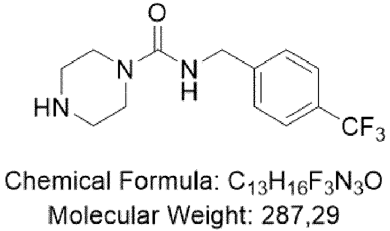

The title compound was prepared from **9** (271 mg, 0.70 mmol, 1.0 eq.) and TFA (0.86 mL mL, 11.2 mmol, 16.0 eq.) following the **general procedure (B).** Compound **10** (108 mg, 0.38 mmol) was obtained as a white solid in 54% yield. **Rf=** 0.15 (CH₂Cl₂/MeOH, 9:1). **¹H NMR (250 MHz, DMSO-*d₆*):** δ 7.64 (dd, *⁴J* = 1.9 Hz, *³J* = 8.5 Hz, 2H, Hₐᵣ), 7.54 (dd, *⁴J* = 1.4 Hz, *³J* = 8.5 Hz, 2H, Hₐᵣ), 4.48 (s, 2H, CH₂), 3.59 (m, 4H, piperazine), 3.37 (m, 4H, piperazine). **LC-MS (ESI⁺)** *m*/*z* = 288.3 [M+H]⁺.

### tert-Butyl-(1-((4-(trifluoromethyl)phenyl)carbamoyl)piperidin-4-yl)carbamate (11)

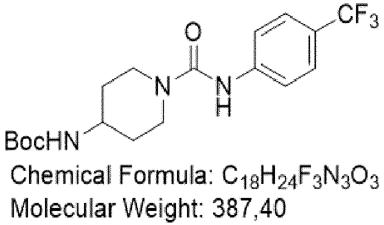

The title compound was prepared from 4-(*N*-Boc-amino)piperidine (431 mg, 2.15 mmol, 1.0 eq.) and 4-(trifluoromethyl)phenyl isocyanate (0.34 mL, 2.36 mmol, 1.1 eq.) following the **general procedure (A).** Compound **11** (809 mg, 2.09 mmol) was obtained as a white solid in 97% yield. **Rf=** 0.40 (CH₂Cl₂/MeOH, 96:4). **¹H NMR (400 MHz, MeOD):** δ 7.57 (d, *³J* = 8.2 Hz, 2H, Hₐᵣ), 7.26 (d, *³J* = 8.2 Hz, 2H, Hₐᵣ), 3.94 (m, 1H, CH-piperidine), 3.43-3.27 (m, 4H, CH₂-piperidine), 1.77-1.94 (m, 4H, CH₂-piperidine), 1.43 (s, 9H, -Boc). **LC-MS (ESI⁺)** *m*/*z* = 388.4 [M+H]⁺. The spectroscopic data are in accordance with those reported in literature (CAS # 1299460-88-0).

### 4-amino-N-(4-(trifluoromethyl)phenyl)piperidine-1-carboxamide (12)

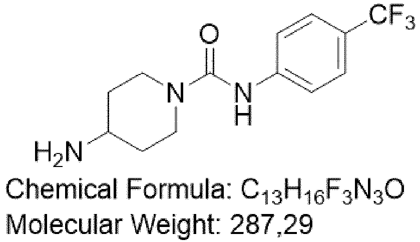

The title compound was prepared from **11** (271 mg, 0.70 mmol, 1.0 eq.) and TFA (0.86 mL mL, 11.2 mmol, 16.0 eq.) following the **general procedure (B).** Compound **12** (138 mg, 0.48 mmol) was obtained as a white solid in 68% yield. **Rf=** 0.26 (CH₂Cl₂/MeOH, 8:2). **¹H NMR (400 MHz, MeOD):** δ 7.57 (d, *³J* = 8.2 Hz, 2H, Hₐᵣ), 7.26 (d, *³J* = 8.2 Hz, 2H, Hₐᵣ), 3.94 (m, 1H, CH-piperidine), 3.43-3.27 (m, 4H, CH₂-piperidine), 1.97-1.77 (m, 4H, CH₂-piperidine). **LC-MS (ESI⁺)** *m*/*z* = 288.3 [M+H]⁺. The spectroscopic data are in accordance with those reported in literature (CAS # 1291448-29-7).

### 4-((2-(pyridin-2-yl)-1H-benzo[d]imidazol-5-yl)methyl)-N-(4-(trifluoromethyl)benzyl)piperazine-1-carboxamide (II-10)

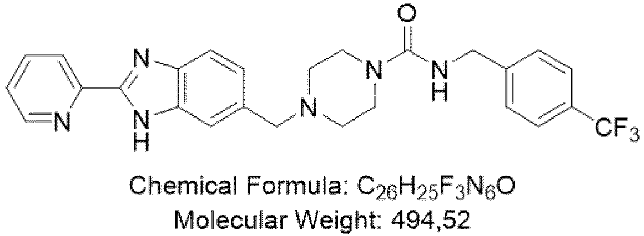

The title compound was prepared from **3** (200 mg, 0.89 mmol, 1.0 eq.) and **10** (256 mg, 0.89 mmol, 1.0 eq.) following the **general procedure (C).** Compound **II-10** (238 mg, 0.48 mmol) was obtained as an orange solid in 54% yield. **Rf=** 0.26 (CH₂Cl₂/MeOH, 9:1). **Mp=** 122 ± 2 °C. **IR (cm⁻¹):** 1621, 1533, 1446, 1403, 1324, 1261, 1159, 1109, 1065. **¹H NMR (400 MHz, Acetone-*d₆*):** δ 12.04 (br s, 1H, NHₐᵣ), 8.66 (d, *³J* = 4.7 Hz, 1H, Hₐᵣ), 8.39 (d, *³J* = 7.9 Hz, 1H, Hₐᵣ), 7.96 (td, *³J* = 1.6 Hz, *³J* = 7.7 Hz, 1H, Hₐᵣ), 7.71-7.56 (m, 4H, Hₐᵣ), 7.51 (d, *³J* = 8.0 Hz, 2H, Hₐᵣ), 7.45 (m, 1H, Hₐᵣ), 7.26 (m, 1H, Hₐᵣ), 6.56 (t, *³J* = 5.6 Hz, 1H, CO-NH), 4.45 (d, *³J* = 5.6 Hz, 2H, N-CH₂), 3.64 (s, 2H, CH₂), 3.46 (m, 4H, CH₂-piperazine), 2.46 (m, 4H, CH₂-piperazine). **¹³C NMR (100 MHz, Acetone-*d₆*):** δ 157.5 (quat C), 151.3 (quat C), 149.3 (quat C, CH), 149.1 (quat C), 146.1 (quat C), 137.1 (CH, quat C), 133.7 (quat C), 128.0 (quat C), 127.9 (4x CH), 124.9 (2x CH), 124.7 (CH), 124.4 (CH), 121.2 (CH), 119.2 (CH), 112.0 (CH), 63.0 (CH₂), 52.8 (2x CH₂, Cₚᵢₚₑᵣ.), 43.9 (2x CH₂, C_{piper.}), 43.6 (CH₂). **HRMS (ESI⁺)** calcd. for C₂₆H₂₆F₃N₆O, 495.2115 [M+H]⁺; found, 495.2121.

### 4-(((2-(pyridin-2-yl)-1H-benzo[d]imidazol-5-yl)methyl)amino)-N-(4-(trifluoromethyl)phenyl) piperidine-1-carboxamide (II-11)

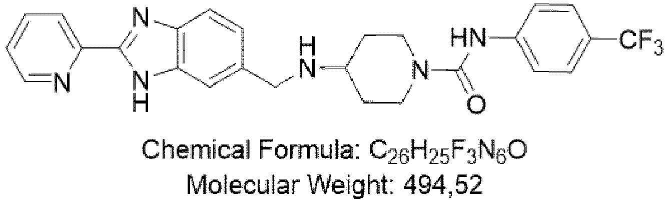

The title compound was prepared from 3 (200 mg, 0.89 mmol, 1.0 eq.) and **12** (256 mg, 0.89 mmol, 1.0 eq.) following the **general procedure (C).** Compound **II-11** (75 mg, 0.15 mmol) was obtained as an orange solid in 17% yield. **Rf=** 0.30 (CH₂Cl₂/MeOH, 9:1). **Mp=** 161 ± 2 °C. **IR (cm⁻¹):** 1705, 1645, 1593, 1516, 1446, 1416, 1366, 1308, 1276, 1240, 1173, 1147, 1103. **¹H NMR (400 MHz, MeOD):** δ 8.75 (d, *³J* = 4.8 Hz, 1H, Hₐᵣ), 8.31 (d, *³J* = 7.9 Hz, 1H, Hₐᵣ), 7.99 (m, 1H, Hₐᵣ), 7.81 (s, 1H, Hₐᵣ), 7.72 (d, *³J* = 8.4 Hz, 1H, Hₐᵣ), 7.62-7.48 (m, 5H, Hₐᵣ), 7.43 (d, *³J* = 8.4 Hz, 1H, Hₐᵣ), 4.27 (s, 2H, CH₂), 3.73 (p, *³J* = 6.8 Hz, 1H, CH-piperidine), 3.24 (m, 2H, CH₂- piperidine), 2.99 (m, 2H, CH₂- piperidine), 2.20 (m, 2H, CH₂- piperidine), 1.61 (m, 2H, CH₂- piperidine). **¹³C NMR (101 MHz, Methanol-*d*₄):** □ 162.0 (quat C), 161.6 (quat C), 155.6 (quat C), 152.2 (quat C), 149.6 (CH), 147.8 (quat C), 143.5 (quat C), 137.2 (CH), 125.4 (quat C), 125.3 (CH), 124.8 (CH), 121.2 (CH), 119.6 (4x CH), 118.2, 54.4 (CH), 49.2 (2x CH₂, C_{piper.}), 42.6 (2x CH₂, C_{piper.}), 29.5 (CH₂). **HRMS (ESI⁺)** calcd. for C₂₆H₂₆F₃N₆O, 495.2115 [M+H]⁺; found, 495.2111.

### Part III. Biological Activity of the compounds

### Methods

Primary macrophage cultures: Murine bone marrow cells were isolated from femurs and cultivated (1 million/mL) for 7 days in Dulbecco's minimal essential medium (DMEM) supplemented with 100 U/mL penicillin and 100 µg/mL streptomycin (Gibco, Australia), 2 mM Lglutamine, 25mM Hepes (Gibco, Australia), 20% horse serum and 30% L929 cell-conditioned medium as source of M-CSF. After further three days in fresh medium, the bone marrow-derived macrophages (BMDM; 10⁵ cells/well) in DMEM supplemented with 100 U/mL penicillin and 100 µg/mL streptomycin, 2 mM L-glutamine, 25mM Hepes and 0.1% FCS were incubated with the compounds. The lyophilised compounds were solubilised in DMSO and used at a non-cytotoxic, 1%-0.3% DMSO final concentration in DMEM supplemented with 100 U/mL penicillin and 100 µg/mL streptomycin, 2 mM L-glutamine, 25mM Hepes.

Absence of cytotoxicity was controlled using MTT incorporation.

The compounds were tested as agonists at concentrations of 1, 3, 10 or 14µM, as compared with reference STING agonist cGAMP (1-14 µM, InvivoGen, San Diego, CA, USA) or DMSO vehicle controls.

For testing their antagonist activity, the compounds were tested at 1, 3, or 10µM, and compared with reference STING antagonist H151 (3 - 14µM, InvivoGen, San Diego, CA, USA) or DMSO vehicle controls , added 3 hr prior to the STING stimuli cGAMP (at 14µM, InvivoGen, San Diego, CA, USA). Cell culture supernatants were harvested after 18h and the concentration of CXCL10 (IP-10) in the supernatant measured by ELISA (R&D System, Minneapolis, USA).

### Results

The compounds were evaluated to determine their activity as STING agonist or antagonist, using as reference compounds commercial STING agonist cGAMP and STING antagonist H151. Murine bone marrow-derived macrophages (BMDM) were stimulated either directly by the compounds, or first with cGAMP in the absence or in presence of the compounds. The secretion of type I interferon (IFN)-induced CXCL10/IP-10 by macrophages was measured. The potential cytotoxic effect was assessed by MTT assay.

The first series of assays was realized on BMDM from control C57/BL6 (B6) mice expressing murine STING sharing 70% homology with human STING (hSTING), to preselect biological active structures. While cGAMP induced CXCL10 secretion at concentrations 3 to 14 µM, the compounds did not induce CXCL10 response at concentrations up to 10-14 µM. The STING antagonist H151 at 2.5 µM inhibited by ca 80% the induction of CXCL10 in response to cGAMP, and several compounds reduced cGAMP-induced CXCL10 response at concentrations up to 1-2.5 µM. The IC50_{cGAMP} of the different compounds are summarized in Table 1. Thus, the compounds are not cytotoxic, and displayed no activity as STING agonist but they displayed some activity as antagonist of murine STING.

A specificity assay is then realized on control B6 BMDM activated by Poly I-C, a TLR3 agonist independent of STING also activating downstream IRF3. Poly I-C induced CXCL10 secretion at concentrations 1 µM. STING antagonist H151 that inhibited 50% of CXCL10 induction in response to cGAMP with an IC50_{cGAMP} 1.67µM, was less potent to inhibit CXCL10 induction in response to Poly I-C, with an IC50_{polyl:C} 8 µM. The high IC50_{polyl:C}, as compared to low IC50_{cGAMP} is a measure of the specificity of the compound for STING pathway. Another reference STING antagonist SN011 inhibited 50% of CXCL10 induction in response to cGAMP with an IC50_{cGAMP} 0.81µM, and was unable to inhibit CXCL10 induced by Polyl:C, with an IC50_{polyl:C} >14 µM, showing a strong specificity for STING. Our compounds I-40, I-52, II-1, II-2, II-3, II-4, I-48, II-6, II-10 and II-11 showed an interesting differential between their capacity to inhibit CXCL10 induced by cGAMP vs Polyl:C, with IC50_{cGAMP} 3-4-fold lower than IC50_{polyl:C}. Thus, the compounds displayed some specificity as antagonist of STING pathway. The IC50 of the different compounds are summarized in Table 1.

To confirm that compounds able to inhibit BMDM activated by Poly I:C, did so independently of STING, we next used BMDM derived from mice genetically deleted for STING. Poly I-C induced CXCL10 secretion at concentrati ons 1 µM in STING^{-/-} BMDM. Reference STING antagonists H151 and SN011 poorly inhibited CXCL10 induction in response to Polyl:C in STING^{-/-} BMDM, with IC50_{polyl:C} 6.31 µM and >14 µM, respectively, confirming their specific for STING. However, compound I-24 which had an IC50_{cGAMP} 1.22 µM was less specific, with an IC50_{polyl:C} 5.84 µM and a IC50_{polyl:C} 3.32 µM in STING^{-/-} BMDM, indicating that it is completely independent of the presence of functional STING, thus that compound I-24 does not act as a STING antagonist.

Finally, the capacity of the compounds to inhibit human STING was tested in BMDM derived from mice expressing human STING in place of their endogenous murine STING (hSTING KI). Interestingly, the reference STING antagonists H151 and SN011 poorly inhibited CXCL10 induction in response to cGAMP in hSTINGKI BMDM, with IC50_{cGAMP} 5.51_µM and >14 µM, respectively, indicating their poor activity on human STING. This is reminiscent of a problem encountered in the development of some STING agonists DMXAA (5,6-dimethylxanthenone-4-acetic acid) and related flavonoid compounds which showed promising preclinical data in rodents but lacked activity on human STING (Kim S et al., 2013). In contrast, our compounds I-52, II-1, II-2, II-3, II-4, I-48, II-6, II-10 and II-11 showed a good activity on human STING with IC50_{cGAMP} of 0.9 µM for compound I-52, IC50_{cGAMP} 1.51 µM for II-3 or IC50_{cGAMP} 2.1 µM for II-4, indicating their strong activity on human STING.

To confirm the activity of the compounds to inhibit the pathway of type I IFN-induced CXCL10/IP-10 secretion by macrophages, the production of type I IFNβ itself was measured. The reference STING antagonist H151 inhibited cGAMP-induced release of IFNβ by B6 BMDM with an IC50 1.7 µM, thus similar to IC50_{cGAMP} 1.67 µM for CXCL10 production. The compounds I52, II-11 and II-8 had IC50 1.04, 1.01 and 1.9 µM, respectively (Table 2), thus confirming their inhibitory activity for type I IFN response.

**Table 1. Summary IC50 (µM): IP-10 production**

| Compound | B6 & cGAMP | B6 & Poly I:C | mSTING KO & Poly I:C | hSTING KI & cGAMP |
|---|---|---|---|---|
| H-151 | 1.67+/-0.16 | 8.03+/-0.72 | 6.31+/-0.40 | 5.51+/-0.87 |
| SN-011 | 0.81 +/- 0.14 | >14µM | >14µM | >14µM |
| **I-24** | 1.22+/-0.08 | 5.84+/-0.3 | 3,32+/-1.74 | not evaluated |
| **I-40** | 1.52+/-0.07 | 6.67+/-0,16 | 5.69+/-0.49 | not evaluated |
| **I-52** | 1.23+/-0.1 | 5.65+/-0,31 | 4.49+/-0.18 | 0.90+/-0.18 |
| **II-1** | 3.30 +/- 0.22 | 9.53 +/- 1,21 | >10 µM | 2.66 +/- 0.33 |
| **II-2** | 3.34 +/- 0.17 | 7.33 +/- 0,65 | 7.69 +/- 1.21 | 2.56 +/- 0.43 |
| **II-3** | 1.69+/-0.29 | 6.81+/-0,36 | 7.68+/-0.67 | 1.51+/-0.17 |
| **II-4** | 2.23+/-0.55 | >10µM | 8,91+/-0,95 | 2.12+/-0.08 |
| **II-5** | >10µM | not evaluated | not evaluated | not evaluated |
| **I-48** | 3.26+/-0.76 | >10µM | >10µM | 2.61+/-0.1 |
| **II-6** | 3.21+/-0.94 | >10µM | >10µM | 2.79+/-0.4 |
| **II-8** | 5.61 +/- 0.12 | >10µM | >10µM | 5.78 +/- 0.77 |
| **II-9** | >10µM | not evaluated | not evaluated | not evaluated |
| **II-7** | >10µM | not evaluated | not evaluated | not evaluated |
| **II-10** | 2.76 +/- 0.17 | 10.33 +/- 1.21 | >10 µM | 2.35 +/- 0.25 |
| **II-11** | 2.57 +/- 0.14 | 7.52 +/- 0.26 | 6.43 +/- 0.16 | 3.19 +/- 0.24 |

Comparative studies are reported with compounds H-151 and SN-011 whose formula are shown on figure 1.

**Table 2. Summary IC50 (µM): IFNβ production**

| Compounds | B6 & cGAMP |
|---|---|
| H-151 | 1.70 +/- 0.12 |
| **I-52** | 1.04 +/- 0.24 |
| **II-11** | 1.01 +/- 0.21 |
| **II-8** | 1.90 +/- 0.53 |

## Claims

1. Compound of formula (I): 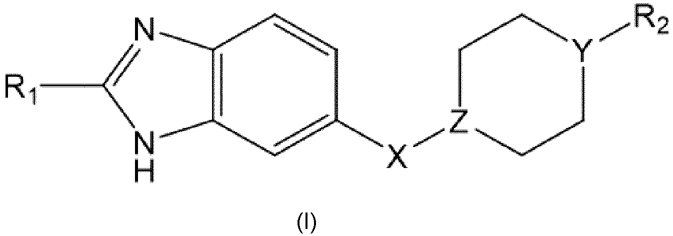 in which:
▪ X represents:
∘ a C=O group;
∘ an alkyl group, linear or branched, comprising 1 to 6 carbon atoms ; or
∘ a group of formula -NR-(C₁-C₆)alkyl-, the alkyl being linear or branched;
▪ Y and Z represents independently a CH or a nitrogen atom;
▪ R₁ represents:
∘ an aryl group, comprising 6 to 10 carbon atoms, non-substituted or substituted by at least one group chosen among: haloalkyl, haloalkoxyl, alkyl or alkoxyl group, the alkyl being linear or branched, and comprising 1 to 6 carbon atoms;
∘ an heteroaryl group, comprising 6 to 10 members, non-substituted or subsituted by at least one group chosen among: haloalkyl, haloalkoxyl, alkyl or alkoxyl group, the alkyl being linear or branched, and comprising 1 to 6 carbon atoms;
∘ a CH-(aryle)₂ group, the aryl comprising 6 to 10 carbon atoms, non-substituted or subsituted by at least one group chosen among: haloalkyl, haloalkoxyl, alkyl or alkoxyl group, the alkyl being linear or branched, and comprising 1 to 6 carbon atoms; or
∘ a CH-(heteroaryl)₂ group, the heteroaryl comprising 6 to 10 members, non-substituted or subsituted by at least one group chosen among: haloalkyl, haloalkoxyl, alkyl or alkoxyl group, the alkyl being linear or branched, and comprising 1 to 6 carbon atoms;
▪ R₂ represents :
∘ a (C₁-C₆)alkyl-aryl-R₃ group, the aryl comprising 6 to 10 carbon atoms and the alkyl being linear or branched;
∘ a C(O)N(R)-aryl-R₅ group, the aryl comprising between 6 to 10 carbon atoms;
∘ a C(O)-(C₁-C₆)alkyl-O-aryl-Hal group, with Hal represents a halogen, the aryl comprising between 6 and 10 carbon atoms and the alkyl being linear or branched;
∘ a C(O)O-(C₁-C₆)alkyl-aryl group, the aryl comprising between 6 to 10 carbon atoms and the alkyl being linear or branched;
∘ a O-aryl-R₃ group, the aryl comprising between 6 to 10 carbon atoms;
∘ a C(O)N(R)-(C₁-C₆)alkyl group, the alkyl being preferably substituted by a -C(=O)O-R' function; or
∘ a C(O)N(R)-(C₁-C₆)alkyl-aryl group, the aryl comprising between 6 to 10 carbon atoms;
▪ R₃ represents a OR₄ group, haloalkyl or haloakoxyl, wherein the alkyl is linear or branched, and comprises 1 to 6 carbon atoms;
▪ R₄ represents a (C₁-C₆)alkyl-aryl-COOR group, a haloalkyl group or a (C₁-C₆)alkyl-aryl-(C₁-C₆)alkyl-PO(OR)₂ group, wherein alkyl is linear or branched and comprises 1 to 6 carbon atoms and the aryl comprises between 6 and 10 carbon atoms;
▪ R₅ represents an alkyl group, linear or branched, comprising 1 to 6 carbon atoms; or a haloalkyl, linear or branched, comprising 1 to 6 carbon atoms;
▪ R or R' represents independently an alkyl group, linear or branched, comprising 1 to 6 carbon atoms, or a hydrogen atom;
or their pharmaceutically acceptable salts, hydrates, solvates, esters or their optical isomers, racemates, diastereoisomers, enantiomers, tautomers, or a mixture thereof;
for use in the treatment of at least one disorder selected among cardiovascular disorders, neurological disorders, autoimmune and inflammatory diseases.

2. Compound according to claim 1 for which R₁ is chosen among:
▪ a phenyl group, non-substituted or substituted by at least one group chosen among: haloalkyl, haloalkoxyl, alkyl or alkoxyl group, the alkyl being linear or branched, and comprising 1 to 6 carbon atoms;
▪ a pyridine group non-substituted or substituted by at least one group chosen among: haloalkyl, haloalkoxyl, alkyl or alkoxyl group, the alkyl being linear or branched, and comprising 1 to 6 carbon atoms;
▪ a pyrimidine group, non-substituted or substituted by at least one group chosen among: haloalkyl, haloalkoxyl, alkyl or alkoxyl group, the alkyl being linear or branched, and comprising 1 to 6 carbon atoms; or
▪ a benzhydryl group non-substituted or substituted by at least one group chosen among: haloalkyl, haloalkoxyl, alkyl or alkoxyl group, the alkyl being linear or branched, and comprising 1 to 6 carbon atoms.

3. Compound according to any one of claims 1 or 2 for which R₁ is chosen among:
▪ a phenyl group non-substituted or substituted by at least one group chosen among: fluoroalkyl, fluoroalkoxyl, alkyl or alcoxyl group, the alkyl being linear or branched, and comprising 1 to 6 carbon atoms;
▪ a pyridine group non-substituted or substituted by at least one group chosen among: haloalkyl, haloalkoxyl, alkyl or alkoxyl group, the alkyl being linear or branched, and comprising 1 to 6 carbon atoms; or
▪ a benzhydryl group non-substituted or substituted by at least one group chosen among: fluoroalkyl, fluoroalkoxyl, alkyl or alcoxyl group, the alkyl being linear or branched, and comprising 1 to 6 carbon atoms.

4. Compound according to any one of claims 1 to 3 for which X represents:
∘ a (C=O) group;
∘ an alkyl group, linear or branched, comprising 1 to 3 carbon atoms; or
∘ a group of formula -NH-(C₁-C₃)alkyl-, the alkyl being linear or branched.

5. Compound according to any one of claims 1 to 4 for which Z represents a CH group and Y represents a nitrogen atom.

6. Compound according to any one of claims 1 to 4 for which Y and Z represents a nitrogen atom.

7. Compound according to any of claims 1 to 4, said compound being selected among one of the followings:

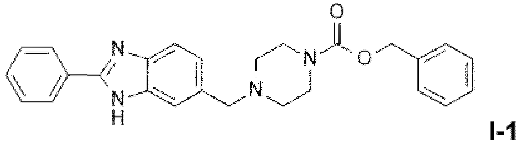

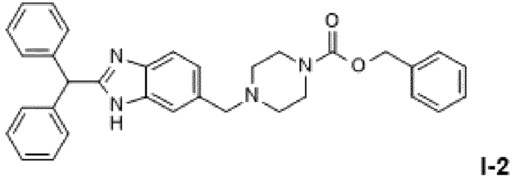

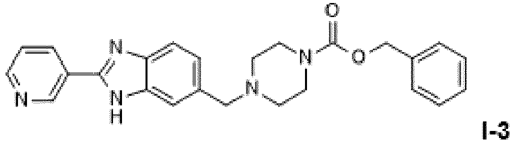

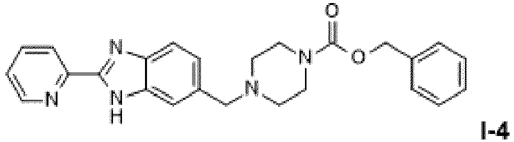

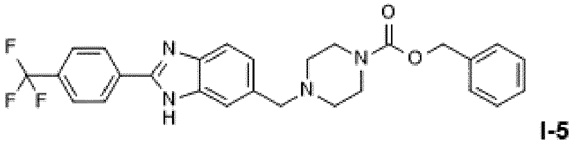

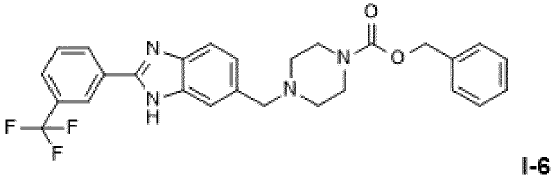

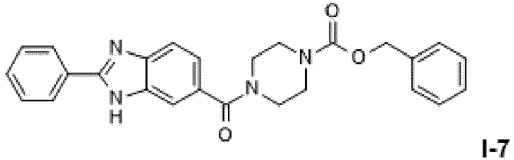

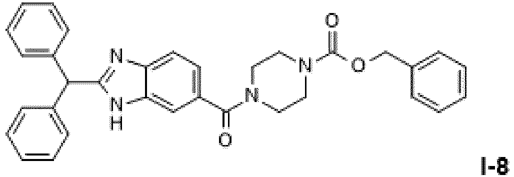

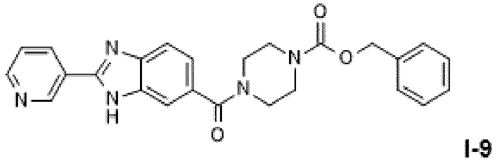

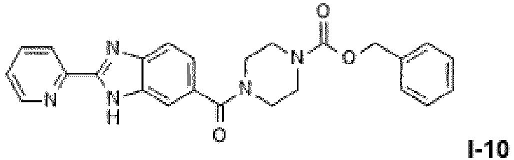

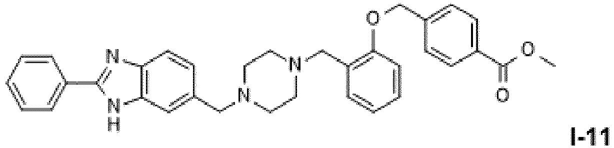

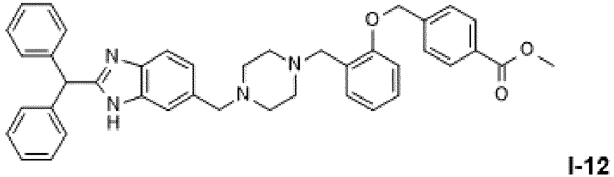

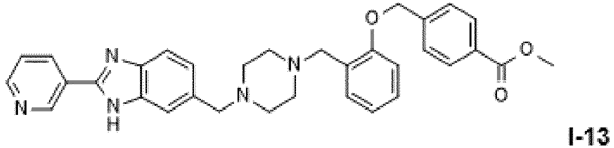

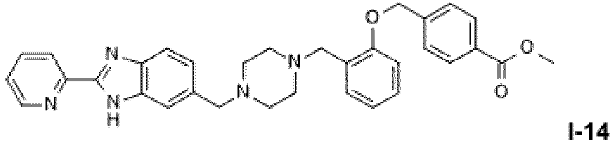

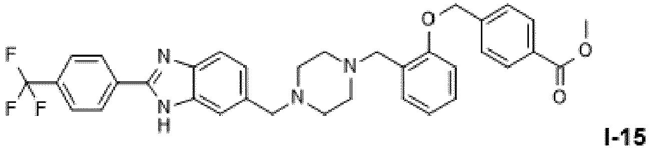

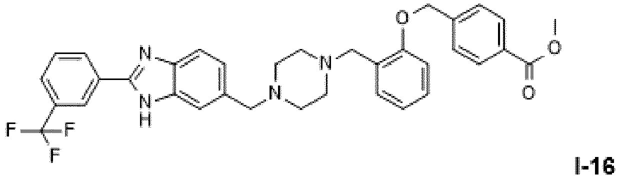

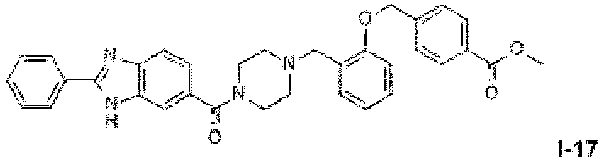

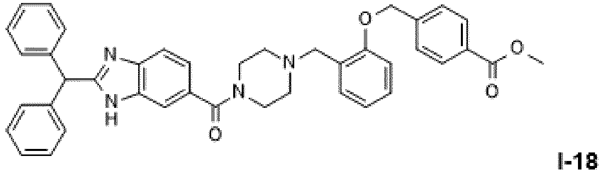

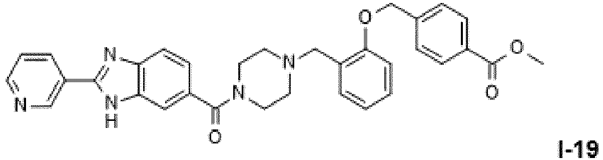

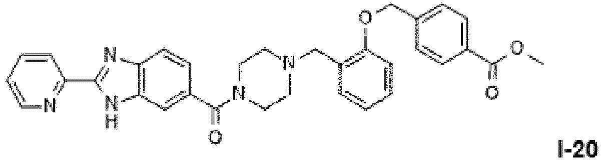

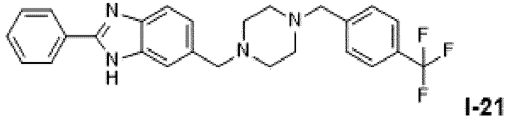

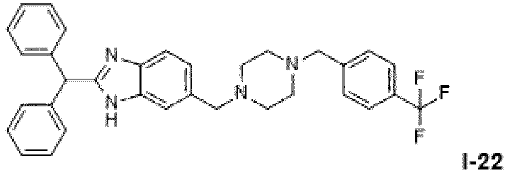

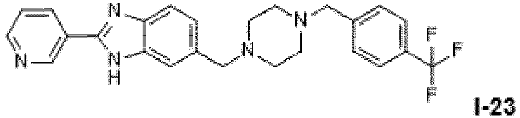

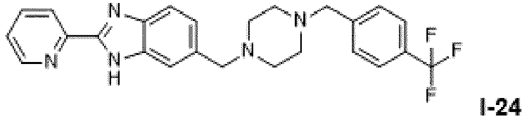

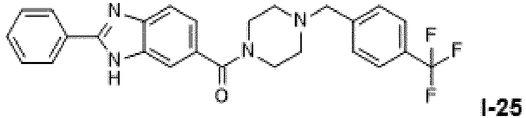

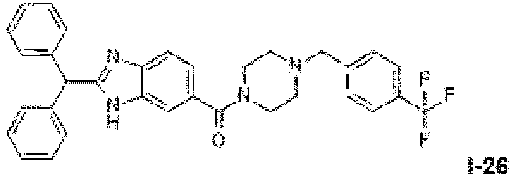

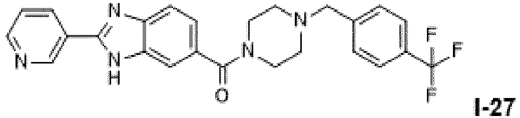

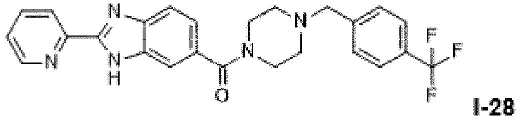

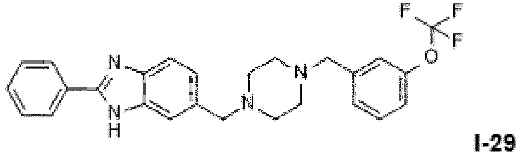

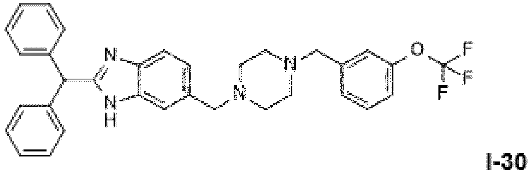

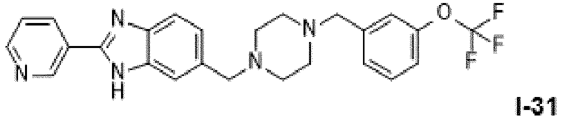

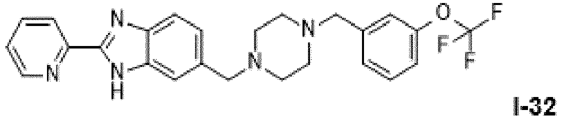

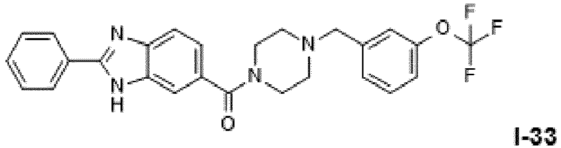

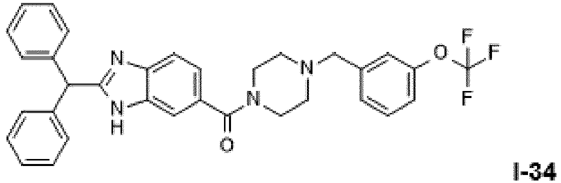

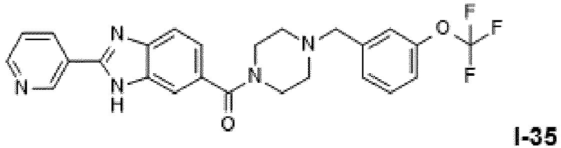

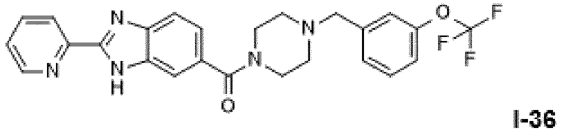

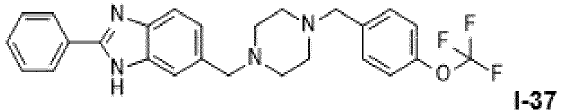

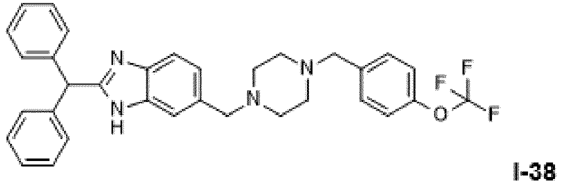

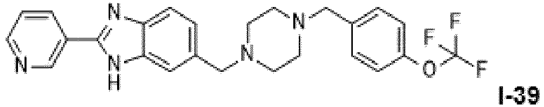

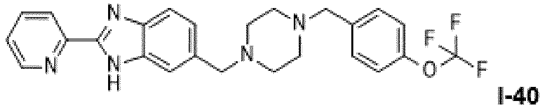

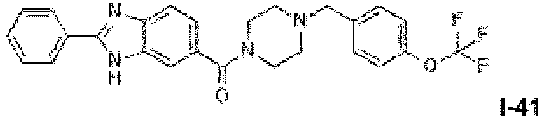

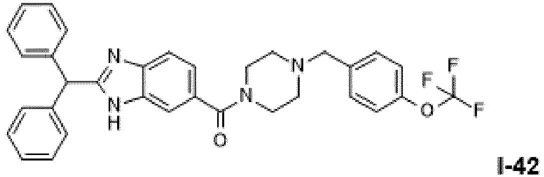

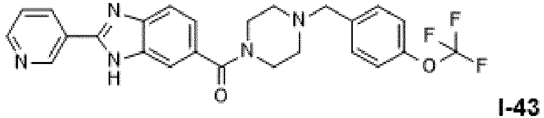

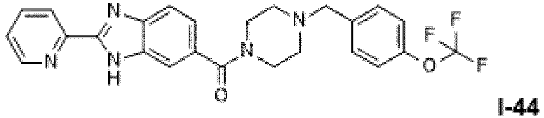

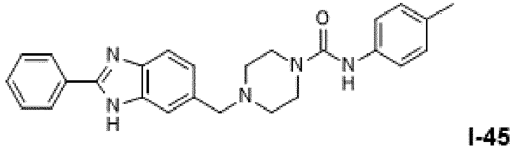

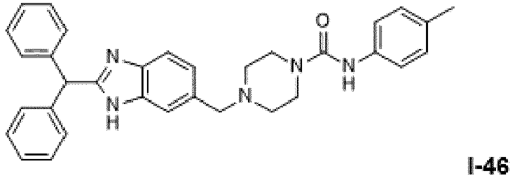

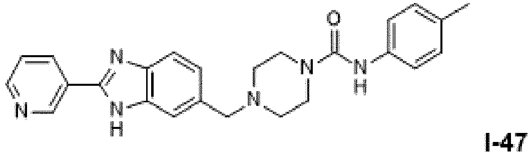

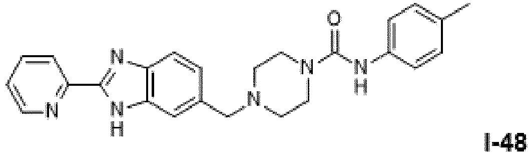

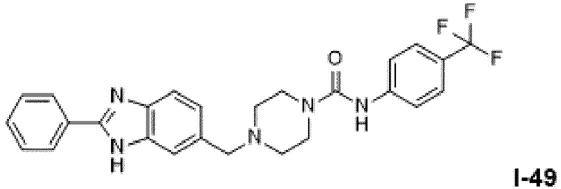

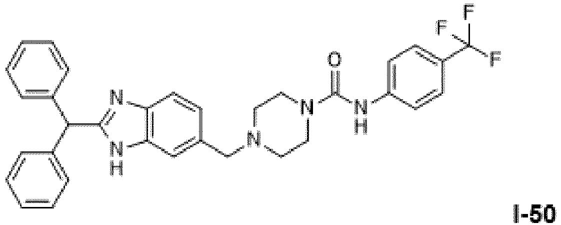

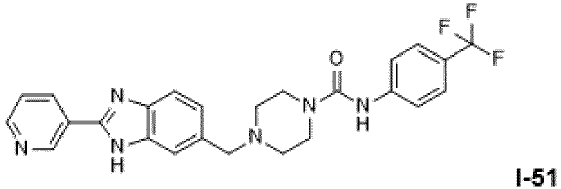

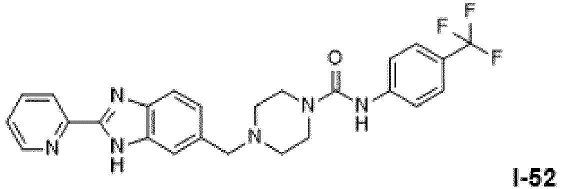

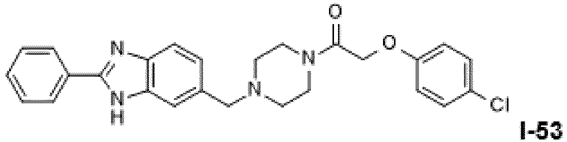

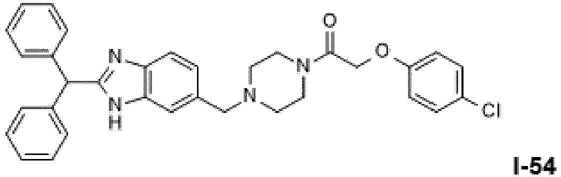

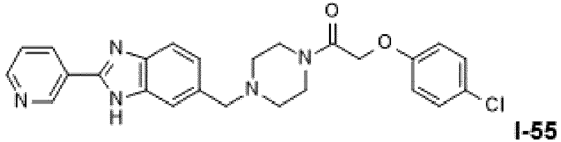

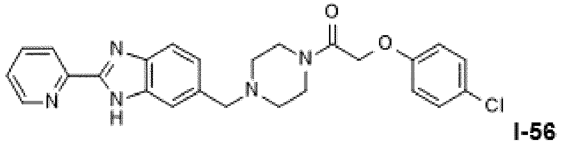

8. Compound of formula (I) according to any of claims 1 to 7, for the treatment of at least one of the autoimmune and inflammatory diseases selected among : interferonopathies such as the so-called Aicardi-Goutières-Syndrome (AGS), or the lupus-like disease such as STING-associated vasculopathy with onset in infancy (SAVI), Familial chilblain lupus; chronic diseases such as lupus, including subtypes of systemic lupus erythematosus (SLE), lupus nephritis (LN), and dermatomyositis, or Sjogren's Syndrome (SS); rheumatoid arthritis; inflammatory bowel disease (IBD) such as ulcerative colitis or Crohn's disease; sepsis; lung inflammatory diseases such as Acute Respiratory Distress Syndrome (ARDS), silicosis, chronic obstructive pulmonary disease (COPD); Metabolic diseases such as Nonalcoholic steatohepatitis (NASH), Alcoholoc liver disease, cirrhosis; Acute pancreatitis; nephropathies, chemo-induced acute kidney injury; chonic inflammation from different origins including viral infection; cancer including Colorectal cancer, Skin cancer, metastasis; senescence and aging-associated inflammation.

9. Compound of formula (I) according to any of claims 1 to 7, for the treatment of at least one of the cardiovascular or neurological disorders, selected among : Myocardial infarction, Chronic heart failure, Endomyocardial fibrosis; Neuroinflammatory diseases such as Parkinson's disease, Alzheimer's disease, Amyotrophic Lateral Sclerosis (ALS), or Age-dependent macular degeneration.

10. Compound of formula (I) according to any of claims 1 to 9, for the treatment of autoimmune diseases involving the cGAS/STING pathway and interferon.

11. Compound of formula (II) : 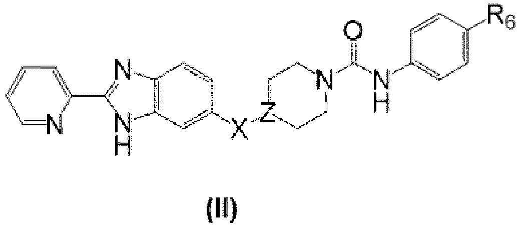
R₆ represents one of the following group selected among:
- a (C₁-C₃)alkyl group preferably substituted by a -C(=O)O-R' function;
- an aryl comprising 6 carbon atoms, said aryl being preferably substituted by a group selected among one of the followings: -CF₃, Cl, NO₂, NH₂, and methyl; and
- a (C₁-C₆)alkyl-aryl group, the alkyl group being preferably -CH₂- and the aryl comprising 6 carbon atoms;
X represents an alkyl group, linear or branched, comprising 1 to 3 carbon atoms; or
a group of formula -NH-(C₁-C₃)alkyl-, the alkyl being linear or branched; and
Z represents a CH or a nitrogen atom
with the exclusion of the following compounds: 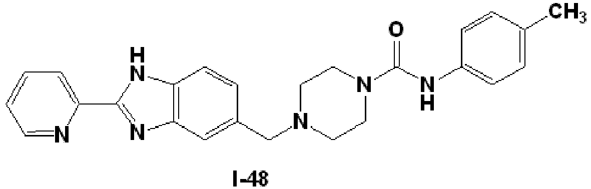 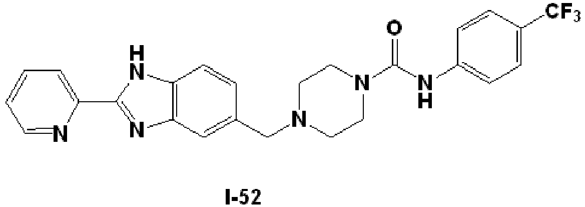
or their pharmaceutically acceptable salts, hydrates, solvates, esters or their optical isomers, racemates, diastereoisomers, enantiomers, tautomers, or a mixture thereof.

12. Compound according to claim 11, said compound having one the following formula:

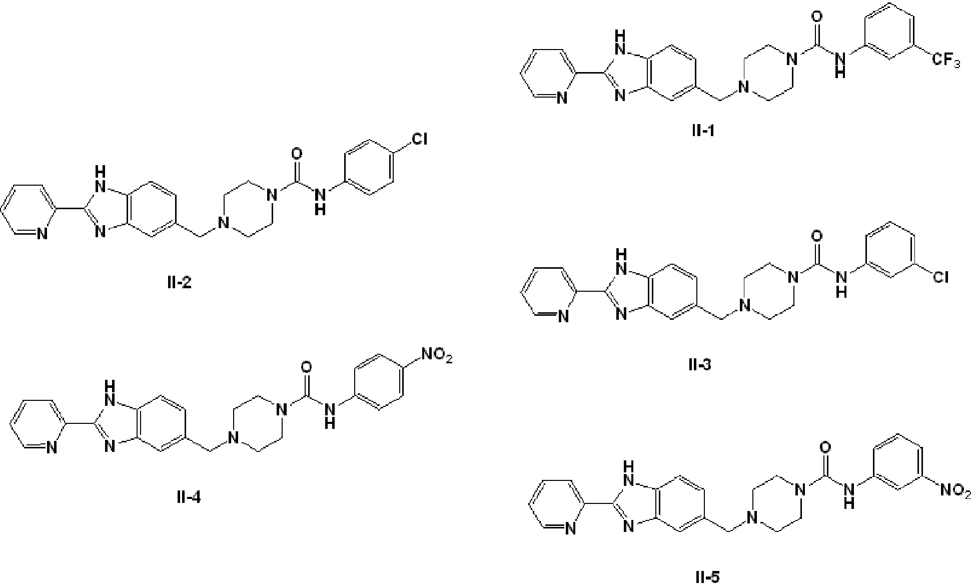

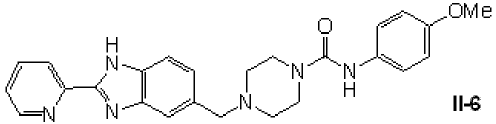

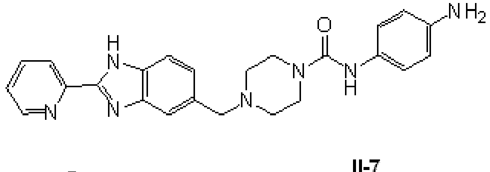

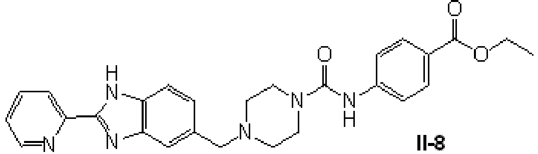

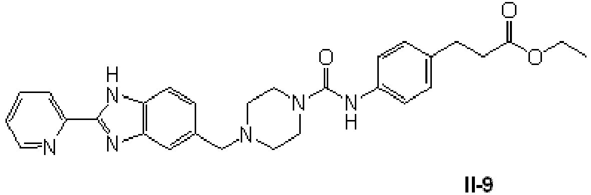

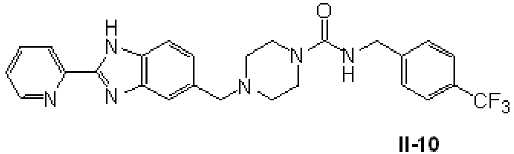

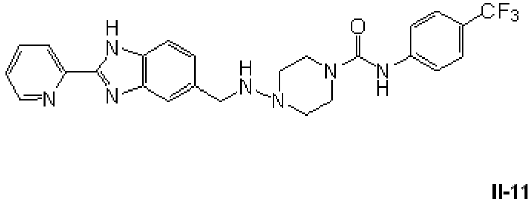

13. A compound according to any of claims 11 or 12, for use as a medicament.

14. Compound according to claim 11 to 12 for use in the treatment of at least one disorder selected among cardiovascular disorders, neurological disorders, autoimmune and inflammatory diseases.

15. Pharmaceutical composition comprising at least one compound of formula (II) according to any one of claims 11 or 12 as active principle, and optionally a pharmaceutically acceptable excipient.
